# EUROPEAN PATENT SPECIFICATION

(11) **EP 0 784 629 B1**
(45) Date of publication and mention of the grant of the patent: **28.04.1999**
(21) Application number: 95933489.7
(22) Date of filing: 05.10.1995
(51) Int. Cl.: C07K 5/06, A61K 38/05

(54) **PEPTIDYL COMPOUNDS AND THEIR THERAPEUTIC USE AS INHIBITORS OF METALLOPROTEASES**
PEPTIDISCHE VERBINDUNGEN UND DEREN THERAPEUTISCHE VERWENDUNG ALS METALLOPROTEINASE-INHIBITOREN
COMPOSES DE PEPTIDYLE ET LEUR USAGE THERAPEUTIQUE EN TANT QU'INHIBITEURS DES METALLOPROTEASES

(30) Priority: 05.10.1994 GB 9420057; 10.03.1995 GB 9504907; 10.05.1995 GB 9509431
(43) Date of publication of application: 23.07.1997
(73) Proprietor: Darwin Discovery Limited, Cambridge CB4 4WE (GB)
(72) Inventor: MONTANA, John, Chiroscience Ltd., Cambridge CB4 4WE (GB); BAXTER, Andrew, Douglas, Chiroscience Limited, Cambridge CB4 4WE (GB); OWEN, David, Alan, Chiroscience Limited, Cambridge CB4 4WE (GB); WATSON, Robert, John, Chiroscience Limited, Cambridge CB4 4WE (GB); PHILLIPSON, Neil, Chiroscience Limited, Cambridge CB4 4WE (GB)
(74) Representative: Perry, Robert Edward
(86) International application number: PCT/GB95/02362
(87) International publication number: WO 96/11209

(56) References cited:
- WO-A-95/13289
- PEPTIDE, CHEMISTRY AND BIOLOGY. PROC. 12TH AM. PEPT. SYMP., CAMBRIDGE, MASS., JUNE 12-16, 1991, 1992 ESCOM, LEIDEN, pages 791-792, R G ALMQUIST ET AL. 'Development of peptidomimetic inhibitors of a newly isolated atrial peptide-degrading enzyme'
- CHEMICAL ABSTRACTS, vol. 104, no. 9, 3 March 1986 Columbus, Ohio, US; abstract no. 65259b, F FAHRENHOLZ ET AL. 'Photoaffinity labelling of the renal V2 vasopressin receptor. Identification and enrichment of a vasopressin-binding subunit' page 366; & EUR. J. BIOCHEM., vol. 152, no. 3, 1986 pages 589-595,
- JOURNAL OF BIOLOGICAL CHEMISTRY (MICROFILMS), vol. 269, no. 48, 2 December 1994 MD US, pages 30227-30231, N FOTOUHI ET AL. 'Potent peptide inhibitors of stromelysion based on the prodomain region of matrix metalloproteinase'

## Description

### Field of the Invention

This invention relates to a novel class of peptidyl derivatives, to processes for their preparation, and to their use in medicine.

### Background to the Invention

WO-A-9513289 describes compounds which have the property of inhibiting the action of metalloproteinases involved in connective tissue breakdown such as collagenase, stromelysin and gelatinase, and which also inhibit the release of TNF, and also indicates conditions in which such compounds may be useful.

Compounds having similar properties are disclosed by Gearing *et al* (1994), Nature 370:555-557; McGeehan *et al* (1994), Nature 370:558-561, and in WO-A-9320047. All of these reported inhibitors contain a hydroxamic acid zinc binding group. Compounds that inhibit collagenase are disclosed in US-A-4511504 and US-A-4568666. Compounds of related structure that are claimed to inhibit stromelysin (proteoglycanase) are disclosed in US-A-4771037.

### SUMMARY OF THE INVENTION

The invention encompasses novel mercaptoalkylpeptidyl compounds of formula (I) which are useful inhibitors of matrix metalloproteinases and/or TNFα-mediated diseases, including degenerative diseases (such as defined in WO-A-9513289) and certain cancers.

As appreciated by those of skill in the art, the significant proportion of homology between enzymes that inhibit matrix metalloproteinases (MMPs) and TNF release leads to the possibility that a compound that inhibits one enzyme may to some degree inhibit these new enzymes. Therefore, inhibitors encompassed in this invention may be useful in the diseases in which these new enzymes are implicated.

In a first aspect of the invention there is provided a compound of general formula (I): 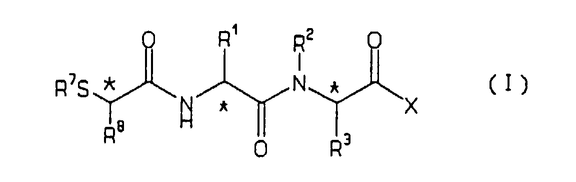 Wherein:
R¹ is C₁₋₆ alkyl, C₂₋₆ alkenyl, -C₁₋₆ alkyl-aryl, aryl, -C₁₋₆ alkyl-heteroaryl, heteroaryl or -C₁₋₆ alkyl-AR⁹ where A represents O,NR⁹ or S(O)ₘ where m=0-2, and R⁹ is H, C₁₋₄ alkyl, aryl, heteroaryl, -C₁₋₄ alkyl-aryl or -C₁₋₄ alkyl-heteroaryl; if A=NR⁹ the groups R⁹ may be the same or different;
R² is H or C₁₋₆ alkyl;
R³ is [Alk]ₙR⁶ where Alk is C₁₋₆ alkyl or C₂₋₆ alkenyl and n is zero or 1;
X is NR⁴R⁵ where either R⁴ is hydrogen or C₁₋₄ alkyl optionally substituted by amino (NH₂), aryl, arylamino, protected amino, di(C₁₋₆ alkyl)amino, mono(C₁₋₆ alkyl)amino, CO₂H, protected carboxyl, carbamoyl, mono(C₁₋₆ alkyl)carbamoyl or di(C₁₋₆ alkyl)carbamoyl, and R⁵ is hydrogen or C₁₋₆ alkyl; or NR⁴R⁵ forms a ring such as pyrrolidino, piperidino or morpholino;
R⁷ is hydrogen or R¹⁰CO where R¹⁰ is C₁₋₄ alkyl, -C₁₋₄ alkyl-aryl, -C₁₋₄ alkyl-heteroaryl, cyclo(C₃₋₆)alkyl, -C₁₋₄ alkyl-cyclo(C₃₋₆)alkyl, C₂₋₆ alkenyl, -C₂₋₆ alkenylaryl, aryl or heteroaryl;
R⁸ is aryl (substituted with R¹¹), heteroaryl (optionally substituted with R¹¹), C₁₋₄ alkyl-R¹¹, -C₁₋₄ alkyl-aryl (substituted with R¹¹), -C₁₋₄ alkyl-heteroaryl (optionally substituted with R¹¹), cyclo(C₃₋₆)alkyl (optionally substituted with R¹¹), cyclo(C₃₋ ₆)alkenyl (optionally substituted with R¹¹), -C₁₋₄ alkyl-cyclo(C₃₋₆)alkyl (optionally substituted with R¹¹), or any of the three groups 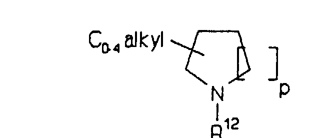 or 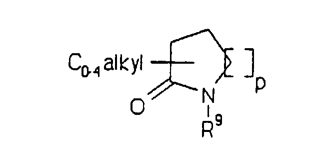 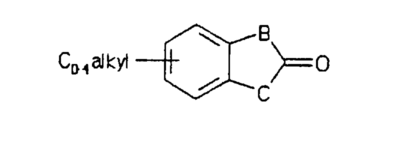
where p is 1 or 2 and B and C are independently selected from O, S, C(R⁹)₂ and NR⁹;
R⁶ is AR⁹, cyclo(C₃₋₆)alkyl, cycle(C₃₋₆)alkenyl, C₁₋₆ alkyl, -C₁₋₆ alkoxy-aryl, benzyloxyaryl, aryl, heteroaryl, -C₁₋₃ alkyl-heteroaryl, -C₁₋₃ alkyl-aryl, -C₁₋₆ alkyl-COOR⁹, -C₁₋₆ alkyl-NHR, CONHR, NHCO₂R, NHSO₂R or NHCOR, R being defined as for R¹⁰;
R¹¹ is SO₂R¹³, SR⁷, SR⁹, COR¹³, N(R⁹)₂, NR⁹R¹², OR⁹, succinimido or phthalimido;
R¹² is H or COR⁹, CO₂R⁹ (where R⁹ is not H), CONHR⁹ or SO₂R⁹ (where R⁹ is not H); and
R¹³ is OH, OC₁₋₄alkyl, O-C₁₋₄ alkyl-aryl, N(R⁹)₂ (in which the R⁹s are the same or different), C₁₋₄ alkyl, aryl, heteroaryl, -C₁₋₄ alkyl-aryl or -C₁₋₄ alkyl-heteroaryl;
   the compound being in the form of a non-salt, salt, solvate or hydrate. Preferred compounds of the invention include those in which, independently or in any combination have:
R¹ is C₁₋₆ alkyl or C₁₋₄ alkylAR⁹ where A is S(O)ₘ ,NR⁹, or O and m=0,1 or 2, and
R⁹ is H, C₁₋₄ alkyl or aryl;
R² is H or C₁₋₄ alkyl;
R³ is [Alk]ₙR⁶ where n=0 or 1, Alk is C₁₋₄ alkyl and R⁶ is C₁₋₄ alkyl, C₁₋₃ alkylaryl, C₁₋₃ alkylheteroaryl or AR⁹;
R⁴ is H;
R⁵ is H or C₁₋₆ alkyl;
NR⁴R⁵ may form a 5-7 membered ring such as a pyrrolidine, piperidine or morpholine;
R⁷ is H or R¹⁰CO where R¹⁰ is C₁₋₄ alkyl;
R⁸ is C₁₋₄ alkylR¹¹, C₁₋₄ alkenylR¹¹, Cyclo(C₃₋₆)alkylR¹¹;
R¹¹ is COR¹³, NR⁹R¹², N(R⁹)₂, succinimido or phthalimido,
R¹² is COR⁹, CO₂R⁹ (provided R9 is not H), or SO₂R⁹ (provided R9 is not H); and R¹³ is OH, OC₁₋₄alkyl or N(R⁹)₂;

Compounds of the invention have IC₅₀ values below 50mM against the MMP enzymes and/or below 50mM in the whole cell assay of TNF inhibition. It will be appreciated that the compounds according to the invention can contain one or more asymmetrically substituted carbon atoms, for example those marked with an asterisk in formula (1). The presence of one or more of these asymmetric centres in a compound of formula (1) can give rise to stereoisomers, and in each case the invention is to be understood to extend to all such stereoisomers, including enantiomers and diastereomers, and mixtures including racemic mixtures thereof. In the formulae herein, the ∼ line is used at a potential asymmetric centre to represent the possibility of *R-* and *S*- configurations, the ...... line and the line to represent a unique configuration at an asymmetric centre.

As used in this specification, alone or in combination, the term "C₁₋₆ alkyl" refers to a straight or branched chain alkyl moiety having from one to six carbon atoms, including for example, methyl, ethyl, propyl, isopropyl, butyl, t-butyl, pentyl, hexyl and the like.

The term "C₁₋₄ alkyl" refers to a straight or branched chain alkyl moiety having from one to four carbon atoms, including for example, methyl, ethyl, propyl, isopropyl, butyl, t-butyl and the like.

The term "C₂₋₆ alkenyl" refers to a straight or branched chain alkyl moiety having two to six carbon atoms and having in addition one double bond, of either E or Z stereochemistry where applicable. This term would include for example, vinyl, 1-propenyl, 1- and 2- butenyl, 2- methyl-2-propenyl etc.

The term "cyclo (C₃₋₆) alkyl" refers to a saturated alicyclic moiety having from three to six carbon atoms and includes for example cyclopropyl, cyclobutyl, cyclopentyl, cyclohexyl and the like.

The term "cyclo (C₃₋₆) alkenyl" refers to an alicyclic moiety having from three to six carbon atoms and having in addition one double bond. This term would include for example cyclopentenenyl or cyclohexenyl.

There term "aryl" means an optionally substituted phenyl or naphthyl group with the substituent(s) being selected, for example, from halogen, trifluoromethyl, C₁₋₆ alkyl, alkoxy, phenyl and the like. The term "halogen" means fluorine, chlorine, bromine or iodine.

The terms "protected amino" and "protected carboxy" mean amino and carboxy groups which are protected in a manner familiar to those skilled in the art. For example, an amino group can be protected by a benzyloxycarbonyl, *tert*-butoxycarbonyl, acetyl or like groups, or in the form of a phthalimido or like group. A carboxyl group can be protected in the form of a readily cleavable ester such as the methyl, ethyl, benzyl or *tert*-butyl ester.

The term "alkoxy" refers to a straight chain or branched chain alkoxy group containing a maximum of six carbon atoms, such as methoxy, ethoxy, propoxy, isopropoxy, butoxy, *tert*-butoxy and the like.

The term "C₀₋₄ alkyl" refers to a bond or straight or branched chain alkyl moiety having from up to four carbon atoms, including for example, methyl, ethyl, propyl, isopropyl and the like.

The term "heteroaryl" refers to aromatic ring systems of five to ten atoms of which at least one atom is selected from the group, O, N or S.

Salts of compounds of formula (I) include pharmaceutically acceptable salts, for example acid addition salts derived from inorganic or organic acids, such as hydrochlorides, hydrobromides, p-toluenesulphonates, phosphates, sulphates, perchlorates, acetates, trifluoroacetates, propionates, citrates, malonates, succinates, lactates, oxalates, tartrates and benzoates.

Salts may also be formed with bases. Such salts include salts derived from inorganic or organic bases, for example alkali metal salts such as magnesium or calcium salts, and organic amine salts such as morpholine, piperidine, dimethylamine or diethylamine salts.

When the "protected carboxy" group in compounds of the invention is an esterified carboxyl group, it may be a metabolically labile ester of formula CO₂R¹⁴ where R¹⁴ may be an ethyl, benzyl, phenethyl, phenylpropyl, α- or β-naphthyl, 2,4-dimethylphenyl, 4-*tert*-butylphenyl, 2,2,2-trifluoroethyl, 1-(benzyloxy)benzyl, 1-(benzyloxy)ethyl, 2-methyl-1-propionyloxypropyl, 2,4,6-trmethylbenzyloxymethyl or pivaloyloxymethyl group.

Compounds of the general formula (I) may be prepared by any suitable method known in the art and/or by the following processes, which itself forms part of the invention.

According to a second aspect of the invention, there is provided a process for preparing a compound of general formula (I) as defined above. It will be appreciated that where a particular stereoisomer of formula (I) is required, the synthetic processes described herein may be used with the appropriate homochiral starting material and/or isomers maybe resolved from mixtures using conventional separation techniques (eg. HPLC).

The compounds according to the invention may be prepared by the following process. In the description and formulae below the groups R¹, R², R³, R⁴, R⁵, R⁶, R⁷, R⁸, R⁹, R¹⁰, R¹¹, R¹², R¹³, R¹⁴, A, B, C and X are as defined above, except where otherwise indicated. It will be appreciated that functional groups, such as amino, hydroxyl or carboxyl groups, present in the various compounds decribed below, and which it is desired to retain, may need to be in protected form before any reaction is initiated. In such instances, removal of the protecting group may be the final step in a particular reaction. Suitable protecting groups for such functionality will be apparent to those skilled in the art. For specific details see "Protective Groups in Organic Synthesis", Wiley Interscience, T W Greene, PGM Wuts. Thus a process for preparing compounds of general formula (I) comprises the steps of:
deprotecting (for example by hydrolysis) a compound of general formula (II) 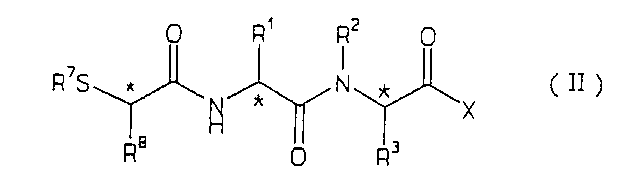 wherein R⁷ represents a suitable protecting group (eg *tert* butyl or acetate).

It will be appreciated that where a particular stereoisomer of formula (I) is required, this may be obtained by conventional resolution techniques such as high performance liquid chromatography. Where desired, however, appropriate homochiral starting materials may be used in the coupling reaction to yield a particular stereoisomer of formula (I). This is exemplified below.

Intermediates of general formula (II) may be prepared by coupling an acid of formula (III) 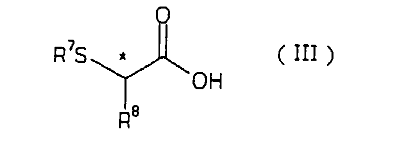 wherein R⁷ and R⁸ are as defined above, or an active derivative thereof, with an amine of formula (IV)

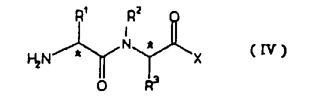

Active derivatives of acids of formula (III) include for example acid anhydrides or acid halides, such as acid chlorides.

The coupling reaction may be performed using standard conditions for amination reactions of this type. Thus, the reaction may be achieved in a solvent, for example an inert organic solvent such as an ether, eg. a cyclic ether such as tetrahydrofuran, an amide eg. a substituted amide such as dimethylformamide, or a halogenated hydrocarbon such as dichloromethane at a low temperature eg. -30°C to ambient temperature, such as -20°C to 0°C, optionally in the presence of as base, eg. an organic base such as an amine, eg. triethylamine or a cyclic amine such as N-methylmorpholine. Where an acid of formula (III) is used, the reaction may additionally be performed in the presence of a condensing agent, for example a diimide such as N,N'-dicyclohexylcarbodiimide, advantageously in the presence of a triazole such as 1-hydroxybenzotriazole. Alternatively, the acid may be reacted with a chloroformate for example ethylchloroformate, prior to reaction with the amine of formula (IV).

The amine of general formula (IV) may be prepared by coupling an acid of formula (V), or an active derivative thereof 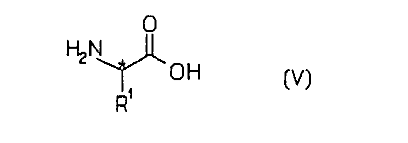 with an amine of formula (VI) 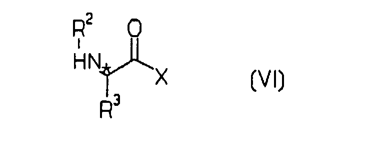 followed by removal of any protecting groups.

Active derivates of acids for formula (V) include for example acid anhydrides or acid halides such as acid chlorides as outlined earlier.

Amino acids and their derivatives as depicted by general formulae (V) and (VI) can be obtained in chiral or racemic form. In the chiral form they provide asymmetric building blocks for the chiral synthesis of compounds of general formula (1). Many of these derivatives can be readily obtained from commercially available starting materials using methods known to those skilled in the art. (See "The Practice of Peptide Synthesis" by M. Bodanszky et al, Springer Verlag, New York, 1984, P. L. Durette, WO92/21360).

As a further extension to the present invention compounds of general formula (II) may be prepared by nucleophilic substitution of compounds of general formula (VII) 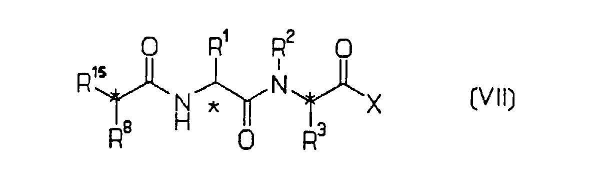 wherein R¹⁵ represents a suitable leaving group (e.g. a halogen such as bromide, or an alkylsulphonate ester such as methanesulphonate) with a thiol of general formula (VIII)

R⁷SH (VIII)

Wherein R⁷ represents a suitable protecting group (eg. *tert*-butyl or acetate), using standard conditions known to those skilled in the art as exemplified in WO 90/05719. Thiols of general formula (VIII) may be obtained from commercially available starting materials using methods known to those skilled in the art. Many thiols of general formula (VIII) are also commercially available.

Compounds of general formula (VII) may be prepared by coupling an acid of general formula (IX) 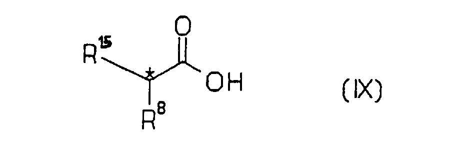 wherein R¹⁵ and R⁸ are as defined above (or suitably protected versions thereof) or an active derivative thereof, with an amine of formula (IV) using similar coupling conditions to those described for the preparation of compounds of formula (II). Carboxylic acids of the structure depicted in formulae (III) and (IX) can be obtained in chiral or racemic form. Many of these derivatives can be readily obtained from commercially available starting materials using methods known to those skilled in the art (see WO 90/05719).

As a further extension to the present invention, intermediates of general formula (II) may be prepared by coupling an acid of formula (X) 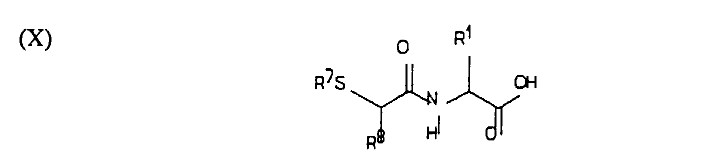 wherein R¹, R⁷ and R⁸ are as defined above, or an active derivative thereof, with an amine of formula (VI) by the procedure described previously.

Acids of general formula (X) may in turn be prepared by coupling an acid of formula (III), or an active derivative thereof with an amine of formula (VI), where X=OH or a suitably protected derivative therof followed by removal of any protecting groups.

Active derivates of acids for formula (V) include for example acid anhydrides or acid halides such as acid chlorides as outlined earlier.

Compounds of formula (I) may also be prepared by interconversion of other compounds of formula (I). Thus, for example, a compound of formula (I) wherein R¹ is a C₁₋₆ alkyl group may be prepared by hydrogenation (using palladium on carbon in suitable solvent, such as an alcohol - eg ethanol) of a compound of formula (I) wherein R¹ is a C₂₋₆ alkenyl group. A further example would include a compound of formula (I) wherein R⁷ is a group R¹⁰ CO may be prepared by acylation (using a suitable acid chloride R¹⁰ COCl, in the presence of a base such as a triethylamine in a suitable solvent, such as a chlorinated solvent - eg dichloromethane) of a compound of formula (I) wherein R⁷ is H.

Any mixtures of final products or intermediates obtained can be separated on the basis of the physico-chemical differences of the constituents, in known manner, into the pure final products or intermediates, for example by chromatography, distillation, fractional crystallization, or by formation of a salt if appropriate or possible under the circumstances.

The compounds according to the invention exhibit *in vitro* inhibiting activities with respect to stromelysin, collagenase and gelatinase. Compounds according to the invention also exhibit *in vitro* inhibition of TNFα release. The activity and selectivity of the compounds may be determined by use of the appropriate enzyme inhibition test, for example as described in Example A hereinafter.

This invention also relates to a method of treatment for patients (including man and/or mammalian animals raised in the dairy, meat or fur industries or as pets) suffering from disorders or diseases which can be attributed to matrix metalloproteinases and/or TNFα as previously described, and more specifically, a method of treatment involving the administration of the matrix metalloproteinase inhibitors of formula (I) as the active constituents.

Accordingly, the compounds of formula (I) can be used among other things in the treatment of osteoarthritis and rheumatoid arthritis, and in diseases and indications resulting from the over-expression of these matrix metalloproteinases such as found in certain metastatic tumour cell lines.

As mentioned above, compounds of formula (I) are useful in human or veterinary medicine since they are active as inhibitors of TNFα and MMPs. Accordingly in another aspect, this invention concerns:
a method of management (by which is meant treatment or prophylaxis) of disease or conditions mediated by TNFα and/or MMPs in mammals, in particular in humans, which method comprises administering to the mammal an effective amount of a compound of formula (I) above, or a pharmaceutically acceptable salt thereof; and a compound of formula (I) for use in human or veterinary medicine, particularly in the management (by which is meant treatment or prophylaxis) of diseases or conditions mediated by TNFα and/or MMPs; and
the use of a compound of formula (I) in the preparation of an agent for the management (by which is meant treatment or prophylaxis) of diseases or conditions mediated by TNFα and/or MMPs.

The disease or conditions referred to above include inflammation, fever, cardiovascular effects, haemorrhage, coagulation and acute phase response, cachexia and anorexia, acute infections, shock states, graft versus host reactions and autoimmune disease; and those involving tissue breakdown such as bone resportion, inflammatory diseases, dermatological conditions, tumour growth, angiogenesis and invasion by secondary metastases, in particular rheumatoid arthritis, osteoarthritis, periodontitis, gingivitis, corneal ulceration, tumour growth, angiogenesis and invasion by secondary metastases.

For the treatment of rheumatoid arthritis, osteoarthritis, and in diseases and indications resulting from the over-expression of matrix metalloendoproteinases such as found in certain metastatic tumour cell lines or other diseases mediated by the matrix metalloendoproteinases or increased TNFα production, the compounds of formula (I) may be administered orally, topically, parenterally, by inhalation spray or rectally in dosage unit formulations containing non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle, sheep, dogs, cats etc, the compounds of the invention are effective in the treatment of humans.

The pharmaceutical composition containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyeryl distearate may be employed. They may also be coated by the techniques described in the US Patents 4,256,108;4,166,452; and 4,265,874 to form osmotic therapeutic tablets for control release.

Formulations for oral use may also be presented as hard gelatin capsules where in the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents may be a naturally occuring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such a polyoxyethylene with partial esters derived from fatty acids and hexitol anhydrides, for example polyoxyethylene sorbitan monooleate. The aqueous suspensions may also contain one or more preservatives, for example ethyl, or n-propyl, p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified, for example sweetening, flavouring and colouring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally- occuring gums, for example gum acacia or gum tragacanth, naturally-occuring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example gycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be in a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of formula (I) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc containing the compounds of Formula (I) are employed. (For purposes of this application, topical application shall include mouth washes and gargles.)

Dosage levels of the order of from about 0.05 mg to about 140 mg per kilogram of body weight per day are useful in the treatment of the above- indicated conditions (about 2.5 mg to about 7 gms per patient per day). For example, inflammation may be effectively treated by the administration of from about 0.01 to 50 mg of the compound per kilogram of body weight per day (about 0.5 mg to about 3.5 gms per patient per day).

The amount of active ingredient that may be combined with the carrier materials to produce a single dosage form will vary depending upon the host treated and the particular mode of administration. For example, a formulation intended for the oral administration of humans may vary from about 5 to about 95 percent of the total composition. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy.

The following Examples 1 to 38 illustrate the invention and their preparation (via the given Intermediates, as appropriate). These and other preferred compounds of the invention are recited in the claims.

In the Examples, the following abbreviations are used:
- RT: Room temperature
- DCC: Dicyclohexylcarbodiimide
- EDC: 1-(3-Dimethylaminopropyl)-3-ethyl carbodiimide hydrochloride

### Intermediate 1 (RS)-2-Bromo-4-methoxycarbonylbutanoic acid

A solution of d-methyl-*D*,*L*-glutamic acid (6.0 g) (preparation according to Hanby *et al,* J. Chem. Soc. (1950), 51:3239) and potassium bromide (15.5 g) in aqueous sulfuric acid (1.25 M, 100 ml) was treated at 0°C portionwise with sodium nitrite (4.0 g) over 1 h. The solution was allowed to warm to RT and was stirred over 2 h, then extracted with ethyl acetate (2 x 100 ml). The combined extracts were dried (MgSO₄) and evaporated *in vacuo* to give the title compound as a colourless oil (4.5 g).
TLC R_{f} 0.14 (25% EtOAc-hexanes)
Similarly prepared were:

### Intermediate 2 (RS)-2-Bromo-5-methoxycarbonylpentanoic acid

From (*RS*)-2-amino-5-methoxycarbonylpentanoic acid (1.8 g) (preparation based on the esterification procedure of Hanby *et al,* J. Chem. Soc. (1950), 51:3239) as a pale brown oil (1.95 g).
TLC R_{f} 0.30 (5% MeOH-CH₂Cl₂)

### Intermediate 3 (RS)-2-Bromo-6-methoxycarbonylhexanoic acid

From (*RS*)-2-amino-6-methoxycarbonylhexanoic acid (3.93 g) (preparation based on the esterification procedure of Hanby *et al,* J. Chem. Soc. (1950), 51:3239) as a colourless oil (4.39 g).
TLC R_{f} 0.26 (5% MeOH-CH₂Cl₂)

### Intermediate 4 (RS)-2-Acetylmercapto-3-methoxycarbonylpropionic acid

A solution of potassium thiolacetate (1.48 g) in methanol (2 ml) was added to a solution of *mono*-methylmaleate (1.64 g) (prepared according the procedure exemplified in J. Am. Chem. Soc. (1986), 108:3) and the mixture was stirred overnight at RT. The solvent was removed by evaporation and the residue was partitioned between water (30 ml) and dichloromethane (30 ml), and the aqueous layer was then acidified to pH3 with 2N aqueous hydrochloric acid. The layers were separated and the aqueous layer was extracted with dichloromethane (2 x 30 ml). The combined organic extracts were dried (MgSO₄) and evaporated *in vacuo* to give a brown oil. Purification by flash column chromatography (eluting with 10% methanol in dichloromethane) gave the title compound as a colourless oil (0.48 g). TLC R_{f} 0.30 (10% MeOH-CH₂Cl₂)

### Intermediate 5 (RS)-2-Acetylmercapto-4-methoxycarbonylbutanoic acid

Potassium thiolacetate (2.0 g) was added to a solution of intermediate 1 (3.0 g) in ethanol (30 ml) and the mixture was stirred at RT overnight. The solution was evaporated *in vacuo* and the residue was partitioned between ethyl acetate (30 ml) and water (30 ml). The organic layer was then washed with saturated brine (30 ml), dried (MgSO₄) and evaporated *in vacuo* to give the title compound as a colourless oil (1.83 g).
TLC R_{f} 0.46 (EtOAc)
Similarly prepared were:

### Intermediate 6 (RS)-2-Acetylmercapto-5-methoxycarbonylpentanoic acid

From Intermediate 2 (1.89 g) as a yellow oil (0.87 g).
TLC R_{f} 0.30 (5 % MeOH-CH₂Cl₂)

### Intermediate 7 (RS)-2-Acetylmercapto-6-methoxycarbonylhexanoic acid

From Intermediate 3 (4.3 g) as a yellow oil (2.78 g).
TLC R_{f} 0.23 (5% MeOH-CH₂Cl₂)

### Intermediate 8 (RS)-(1,1-Dimethylethyl)2,4-dibromobutyrate

Bromine (31.3ml, 0.54mol) was added dropwise over 4h at 100°C to neat stirring 4-bromobutyryl chloride (100g, 0.54mol). The resulting acid chloride was cooled then added dropwise at 0°C to a stirred solution of *tert*-butanol (240ml) and triethylamine (64ml, 0,461mol) in anhydrous dichloromethane (600ml). 2M Hydrochloric acid was added and the layers separated. The organic portion was then washed sequentially with 10% sodium metabisulphite solution (2x500ml), water (500ml) and brine (500ml), dried (MgSO₄) and evaporated *in vacuo* to provide the title compound (120g, 86%) as a brown liquid.
¹H NMR (250MHz; CDCl₃), Ref., TMS) 1.50 (9H, s), 2.45 (2H, q), 3.55 (2H, t) and 4.40 (1H, dd). Similarly prepared were:

### Intermediate 9 (RS)-(1,1-Dimethylethyl) 2,6-dibromohexanoate

From 6-bromohexanoyl chloride (100g, 0.47mol), as a brown oil (133g, 87%). ¹H NMR (250MHz; CDCl₃, Ref., TMS) 1.50 (9H, s), 1.6-2.1 (6H, m), 3.4 (2H, t) and 4.1 (1H, dd).

### Intermediate 10 (RS)-(1,1-Dimethylethyl) 2,4-bis-(acetylmercapto)butyrate

Potassium thiolacetate (1.51g, 13.2mmol)was added to a stirred solution of intermediate 8 (2g, 6.6mmol) in methanol (25ml) and the mixture stirred at RT overnight. The mixture was diluted with dichloromethane (100ml), washed with brine (2x50ml), dried (MgSO₄) and evaporated *in vacuo* to a yellow oil. Purification by flash column chromatography (eluting with 30% dichloromethane in hexane) provided the title compound (1.1g, 57%) as a colourless oil.
TLC R_{f} 0.57 (CH₂Cl₂)
Similarly prepared were:

### Intermediate 11 (RS)-(1,1-Dimetllylethyl)2,6-bis-(acetylmercapto)hexanoate

From Intermediate 9 (2g, 6.32mmol), as a colourless oil (1.09g, 57%)
TLC R_{f} 0.57 (CH₂Cl₂)

### Intermediate 12 (RS)-2,3-Bis-(acetylmercapto)propionic acid

A solution of thiolacetic acid (1.12 g) in 1N aqueous potassium hydroxide (14.7 ml) was added dropwise to a solution of 2,3-dibromopropionic acid (1.71 g) in 1N aqueous potassium hydroxide (7.35 ml) and the mixture was stirred 5 h at RT. The pH of the mixture was adjusted to 8-9 by the addition of further 1N aqueous potassium hydroxide and the mixture was stirred a further 2 h, then acidified to pH1-2 by the addition of concentrated hydrochloric acid and extracted with ethyl acetate (2 x 25 ml). The combined extracts were dried (Na₂SO₄) and evaporated to give a yellow oil. The product from two reactions was combined and purification by flash column chromatography (eluting with 4% acetic acid-toluene) gave the title compound as a colourless oil (0.422 g).
TLC R_{f} 0.15 (5% AcOH-toluene)

### Intermediate 13 (RS)-2,4-Bis-(acetylmercapto)butyric acid

A solution of Intermediate 10 (1.1g, 3.7mmol) in dichloromethane (50ml) was treated with trifluoroacetic acid (2.9ml, 37mmol) and the mixture stirred at RT overnight. Water (50ml) was added and the mixture extracted with dichloromethane (3x40ml). The combined organic extracts were then washed with water (50ml) and brine (50ml), dried (MgSO₄) and evaporated *in vacuo* to provide the product (870mg, 98%) as a pale yellow oil.
TLC R_{f} 0.12 (25 % MeOH-CH₂Cl₂)

### Intermediate 14 5-Phthalimidopentanoic acid

N-Carboethoxyphthalimide (10.96 g) was added in one portion to a vigorously stirred solution of 5-aminopentanoic acid (5.0 g) and sodium carbonate (5.35 g) in water (150 ml) at RT. The mixture was stirred until essentially all the solid material had dissolved (30 min), then it was filtered. The filtrate was acidified to pH1 with 6N aqueous hydrochloric acid (ca. 22 ml) and the white precipitate was collected by filtration and washed thoroughly with water (150 ml). The solid was dried in air, then *in vacuo* to give the title compound as a colourless solid (6.8g).
¹H NMR (250 MHz; CDCl₃, Ref., TMS) d 1.6-1.8 (4H, m), 2.20 (2H, t), 3.85 (2H, t), 7.70-7.75 (2H, m), 7.85-7.95 (2H, m), 10.2 (1H, br s)

### Intermediate 15 2-(3-Phthalimidophenyl)acetic acid

From 2-(3-aminophenyl)acetic acid (3.0g) as an off-white solid (4.0g, 72%).
TLC R_{f} 0.36 (7.5% MeOH-0.5% AcOH-CH₂Cl₂)

### Intermediate 16 (RS) 2-Bromo-5-phthalimidopentanoic acid

Intermediate 14 (5.0 g 20.2 mmol) and thionyl chloride (10 ml) were heated together at 65°C for 30 min. N-Bromosuccinimide (5.4 g) and further thionyl chloride were added, plus 48% aqueous HBr (1 drop). The solution was heated at 60°C for 10 min then 70°C for 2 h 15 min. Further *N*-bromosuccinimide (850 mg) was added and the mixture was heated at 70°C for 2 h. Excess thionyl chloride was removed by evaporation under reduced pressure and the oily residue was diluted with dry tetrahydrofuran (200 ml) and water (200ml). The mixture was then treated cautiously with solid sodium bicarbonate to pH 7-8 then stirred overnight at RT. Excess tetrahydrofuran was removed *in vacuo* and the residue washed with dichloromethane (3x300ml). The aqueous portion was then cautiously acidified to pH 1 using 6M hydrochloric acid and extracted with dichloromethane (4x200ml). The combined extracts were then washed with water (2x400ml) and brine (400ml), dried (MgSO₄) and evaporated *in vacuo* to provide the product (4.7g, 71%) as a fawn solid. TLC R_{f} 0.47 (EtOAc)

### Intermediate 17 (RS) 2-Acetylmercapto-5-phthalimidopentanoic acid

A solution of Intermediate 16 (3.0g, 9.2mmol) in methanol (30ml) was treated with potassium thiolacetate (1.05g, 9.2mmol) and the mixture stirred at RT overnight. The mixture was evaporated *in vacuo,* the residue dissolved in dichloromethane (100ml) then the solution washed with water (2x50ml), dried (MgSO₄) and evaporated *in vacuo* to provide the product (2.4g, 81%) as a pale yellow foam. TLC R_{f} 0.43 (EtOAc)

### Intermediate 18 (RS)-N-[2-Bromo-5-phthalimidopentanoyl]-L-leucyl-L-phenylalanine N-methyl amide

Intermediate 14 (4.0g) and thionyl chloride (2.92 ml) were heated together at 65°C for 30 min. *N*-Bromosuccinimide (2.51 g) and further thionyl chloride were added, plus 48% aqueous HBr (1 drop). The solution was heated at 60°C for 10 min then 70°C for 2 h 15 min. Further *N*-bromosuccinimide (850 mg) was added and the mixture was heated at 70°C for 2 h. Excess thionyl chloride was removed by evaporation under reduced pressure and the oily residue was diluted with dry dichloromethane (10 ml). A portion of the supernatant (4.0 ml) was added to a solution of *L*-leucyl-*L*-phenylalanine N-methylamide (1.18 g) and triethylamine (0.24 ml) in dry dichloromethane (10 ml) at 0°C, and this mixture was stirred overnight at RT. The mixture was diluted with dichloromethane and washed with saturated aqueous sodium bicarbonate solution (50 ml), 1N aqueous hydrochloric acid (50 ml), and saturated brine (50 ml), then dried (MgSO₄) and evaporated in *vacuo* to give a brown solid. This material was purified by flash column chromatography (4 x 18 cm; eluting with 2% methanol-dichloromethane) to give the title compound as a pink solid (1.2 g).
TLC R_{f}0.34 (5% MeOH-CH₂Cl₂)

### Intermediate 19 (RS)-2-[(1,1-Dimethylethyl) mercapto]-5-phthalimidopentanoic acid

*Tert*-butylthiol (11.3ml, 0,1mol) was added to a stirred solution of potassium *tert*-butoxide (22.45g, 0.1mol) in anhydrous tetrahydrofuran (215ml) and the mixture stirred at RT for 20 min. A solution of Intermediate 16 (32.6g, 0.1mol) in anhydrous tetrahydrofuran (80ml) was then added and the mixture stirred at RT overnight. Water (300ml) was added, the mixture acidified to pH 1 with 1N hydrochloric acid and extracted with dichloromethane (3x200ml). The combined extracts were then dried (MgSO₄) and evaporated *in vacuo* to provide a yellow oil. Purification by flash column chromatography (eluting with 5-15% dichloromethane in ethyl acetate) then crystallisation from dichloromethane/hexane furnished the title compound
(22g, 66%) as a pale yellow solid.
TLC R_{f} 0.48 (10% MeOH-CH₂Cl₂)

### Intermediate 20 (RS)-N-[2-[(1,1-Dimethylethyl)mercapto]-5-phthalinfidopentanoyl]-L-leucyl-L-phenylalanine N-methyl amide

A solution of Intermediate 19 (8.06g, 24mmol) and *L*-leucyl-*L*-phenylalanine *N*-methyl amide (7.0g, 24mmol) in dichloromethane (250ml) was treated with *N*-hydroxybenzotriazole (3.9g, 28.9mmol) then EDC (5.06g, 26.4mmol) and the mixture stirred at RT overnight. The mixture was washed with 1N hydrochloric acid (300ml) and the aqueous portion re-extracted with dichloromethane (2x100ml). The combined extracts were washed sequentially with 1N hydrochloric acid (300ml), 8% sodium bicarbonate (300ml), water (300ml) and brine (300ml), dried MgSO₄) and evaporated in *vacuo* to provide a pale yellow solid. Purification by flash column chromatography (eluting with 5% methanol in dichloromethane) provided the title compound (12.8g, 88%) as a near white solid.
TLC R_{f} 0.55 (10% MeOH-CH₂Cl₂)

### Intermediate 21 (RS)-N-[2-[(1,1-Dimethylethyl)mercapto]-5-amnopentanoyl]-L-leucyl-L-phenylalanine N-methyl amide

A solution of Intermediate 20 (2.56g, 4.2mmol) in a mixture of tetrahydrofuran (10ml) and ethanol (50ml) was treated with hydrazine hydrate (10ml, xs) and the mixture heated under reflux for 2h. After cooling to RT water (30ml) was added, the solvent removed in *vacuo,* the residue acidified to pH 1 with 1N hydrochloric acid and washed with dichloromethane (2x100ml). The aqueous layer was then basified to pH 14 with 2M sodium hydroxide and extracted with dichloromethane (2x100ml). The combined extracts were washed with brine (100ml), dried (MgSO₄) and evaporated *in vacuo* to provide a pale yellow solid. Purification by flash column chromatography (eluting with 10-25% methanol in dichloromethane) provided the title compound (9.2g, 91%) as a near white solid.
TLC R_{f} 0.23 (30% MeOH-CH₂Cl₂)

### Intermediate 22 (RS)-N-[2-[(1,1-Dimethylethyl)mercapto]-5-succinimidopentanoyl]-L-leucyl-L-phenylalanine N-methyl amide

Succinic anhydride (0.33g, 3.3 mmol) was added to a stirred solution of Intermediate 21 (1.14g, 2.38mmol) and triethylamine (2ml, 14.4mmol) in anhydrous dichloromethane (45ml), the mixture was then stirred at RT overnight. The mixture was diluted with dichloromethane (75ml) and washed successively with 1N hydrochloric acid (100ml), 8% sodium bicarbonate (100ml), water (100ml) and brine (100ml), dried MgSO₄) and evaporated in *vacuo* to provide a pale yellow solid. Purification by flash column chromatography (eluting with 5% methanol in dichloromethane) provided the title compound (1.2g, 95%) as a near white solid.
TLC R_{f} 0.58 (10% MeOH-CH₂Cl₂)
Similarly prepared was:

### Intermediate 23 (RS)-N-[2-[(1,1-Dimethylethyl)mercapto]-5-(4-pyridylcarbonyl) aminopentanoyl]-L-leucyl-L-phenylalanine N-methyl amide

From Intermediate 21 (2.03g, 4.24mmol) and isonicotinoyl chloride (834mg, 4.66mmol), as a near white solid (1.81g, 77%).
TLC R_{f} 0.22 (10% MeOH-CH₂Cl₂)

### Intermediate 24 (RS)-N-[2-[(2-Nitrophenylsulphenyl)mercapto]-5-succinimido- pentanoyl]-L-leucyl-L-phenylalanine N-methyl amide

2-Nitrophenylsulphenyl chloride (3.26g, 6.26mmol) was added to a stirred solution of Intermediate 22 (618 mg, 1.1 mmol) in glacial acetic acid (75ml) and the mixture stirred at RT overnight. The solvent was removed in *vacuo* and the residue purified by flash column chromatography (eluting with 5% methanol in dichloromethane) to provide the title compound (665 mg, 92%) as a yellow solid.
TLC R_{f} 0.25 (10% MeOH-CH₂Cl₂)
Similarly prepared was:

### Intermediate 25 (RS)-N-[2-[(2-Nitrophenylsulphanyl)mercapto]-5-(4-pyridylcarbonyl)aminopentanoyl]-L-leucyl-L-phenylalanine N-methyl amide

From Intermediate 23 (1.92g, 3.29mmol), as a yellow solid (1.66g, 74%).
TLC R_{f} 0.29 (10% MeOH-CH₂Cl₂)

### Intermediate 26 (R)-N-(Phenylmethoxy)carbonyl-(S-methyl)-L-cysteine

Benzyl chloroformate (11.1 ml, 65mmol) was added dropwise to a stirred solution of (*S*-methyl)-*L*-cysteine (10g, 75mmol) in 2M aqueous sodium hydroxide (50ml) and the mixture stirred at RT for 4h. The mixture was then basified to pH14 with further 2M sodium hydroxide and the solution washed with ethyl acetate (4x50ml). The aqueous phase was acidified to pH3 with concentrated hydrochloric acid and then extracted with ethyl acetate (4x70ml). The combined extracts were washed with brine, dried MgSO₄) and evaporated *in vacuo* to provide the product as a pale yellow oil (16.8g, 84%).
TLC R_{f} 0.21 (10% MeOH-CHCl₃)

### Intermediate 27 (R)-N-(1,1-Dimethylethoxy)carbonyl-(S-methyl)cysteine

Di-tert-butyldicarbonate (8.88g, 40.7mmol) was added to a stirred solution of *S*-methyl-*L*-cysteine (5g, 37mmol) and sodium bicarbonate (7.8g, 92.5mmol) in a mixture of water (150ml) and dioxane (100ml) at 0°C. The mixture was allowed to warm to RT and stirred overnight, then diluted with water (100ml), acidified to pH 3 using 1N hydrochloric acid then extracted with ethyl acetate (3x100ml). The combined extracts were washed with brine, dried (MgSO₄) and evaporated *in vacuo* to provide the title compound as a colourless oil (6.64g, 76%).
TLC R_{f} 0.55 (MeOH-H₂O)
Similarly prepared was:

### Intermediate 28 (S)-N-(1,1-Dimethylethoxy)carbonyl-propylglycine

From (*S*)-norvaline (5g, 42.7mmol), as a colourless oil (6.3g, 68%).
TLC R_{f} 0.50 (MeOH-H₂O)

### Intermediate 29 (RS)-N-Acetyl-[b-(2-pyridyl)]alanine

A solution of sodium methoxide (65.8g, 1.22mol) in methanol (300ml) was treated portionwise with diethylacetamidomalonate (132.3g, 0.61mol) maintaining a temperature of *ca.* 45°C. The mixture was then heated under reflux for 15min. The mixture was cooled to 50°C then treated slowly with a suspension of 2-chloromethylpyridine hydrochloride (100g, 0.61mol), the pink suspension was then heated under reflux for a further 6h. Water (500ml) was added followed by 10M sodium hydroxide (122ml, 1.22mol) and the pH was maintained at *ca.* 11 while heating the mixture at 70°C overnight. The mixture was cooled to RT and the methanol removed *in vacuo.* The aqueous residue was washed with ethyl acetate (2x500ml), then acidified to pH 5 and further washed with ethyl acetate (2x500ml), before evaporating in *vacuo* to provide a semi-solid. The residue was triturated with hot ethanol (500ml) and the sodium chloride removed by filtration. The filtrate was then evaporated *in vacuo* and the residue crystallised from methanol-ethyl acetate to provide the title compound (70g, 66%) as a yellow solid.
TLC R_{f} 0.5 [*n*-BuOH-AcOH-pyridine-H₂O (15-3-10-12)]
Similarly prepared was:

### Intermediate 30 (RS)-N-Acetyl-[b-(4-thiazolyl)]alanine

From 4-(chloromethyl)thiazole, as a white foam (6.7g, 73%). TLC Rf 0.37 (1 % AcOH-20% Hexane-EtOAc)

### Intermediate 31 (S)-(1,1-Dimethylethoxy)carbonyl-[b-(2-pyridyl)]alanine

A suspension of Intermediate 29 (65g, 0.31mol) in aqueous potassium dihydrogen orthophosphate (10mM, 650ml) was warmed to 40°C and the resulting mixture (pH 4) basified to pH 8 with 10M sodium hydroxide (10ml) forming a solution. Acylase 30,000 (1.3g) was added and the mixture incubated at 40°C overnight. The resulting suspension was cooled to RT, the solid removed by filtration, then the filtrate acidified to pH 1 with 2N hydrochloric acid and washed with ethyl acetate (2x300ml). The aqueous portion was evaporated in *vacuo* to provide a semi-solid which was triturated with hot methanol (300ml) and the solid removed by filtration. To the cooled solution was then added 5M sodium hydroxide to pH 10, followed by di-*tert*-butyldicarbonate (28.6g, 0.131 mol), the mixture was maintained at pH 10 by the addition of 5M sodium hydroxide (44ml) over a period of 6h. The methanol was then removed *in vacuo*, the aqueous residue washed with ether (2x500ml) then acidified to pH 3 with 1M potassium hydrogen sulphate. The mixture was extracted with ethyl acetate (4x500ml), the combined extracts washed with brine (500ml), dried MgSO₄) and evaporated *in vacuo* to provide the crude product which was crystallised from ethyl acetate as a white solid (11.6g, 23%).
[a]_{D} -16.0° (c=1, MeOH)
Similarly prepared was:

### Intermediate 32 (S)-N-(1,1-Dimethylethoxy)carbonyl-[b-(4-thiazolyl)]alanine

From Intermediate 30, as a white solid (11g, 94%).
HPLC (Chirex (D)-penicillamine; 2mM CuSO₄; 0.7ml/min; RT=3.25min; 99%ee.

### Intermediate 33 (S)-N-(1,1-Dimethylethoxy)carbonyl[b-(2-pyridyl)]alanine N-methyl amide

DCC (4.07g, 19.7mmol) was added to an ice cooled solution of Intermediate 31 (5.0g, 18.8mmol) and N-hydroxysuccinimide (2.27g, 19.7mmol) in dry tetrahydrofuran (200ml). After stirring at RT for 3h the mixture was treated with 40%w/v aqueous methylamine (6.8ml, 79 mmol) and stirred for a further 2h. The precipitated solid was removed by filtration, the filtrate evaporated *in vacuo* and the residue dissolved in dichloromethane (100 ml). The solution was washed sequentially with water (2x100ml), 8% sodium bicarbonate (2x100ml) and brine (100ml), dried MgSO₄) and evaporated *in vacuo* to provide the crude product. Purification by column chromatography eluting with 2-5 % methanol/dichloromethane provided the title compound (2.7g, 51%) as a red foam.
TLC R_{f} 0.38 (3% MeOH-CH₂Cl₂)
Similarly prepared was:

### Intermediate 34 (S)-N-(1,1-Dimethylethoxy)carbonyl-L-[b-(4-thiazolyl)]alanine N-methyl amide

From Intermediate 32, as a white solid (3.0g, 64%).
TLC R_{f} 0.46 (5% MeOH-CH₂Cl₂)

### Intermediate 35 (R)-N-(1,1-Dimethylethoxy)carbonylpenicillamine N-methyl amide

A solution of (*R*)-*N*-(1,1-dimethylethoxy)carbonylpenicillamine (14g, 56, 1mmol), *N*-hydroxybenzotriazole (7.6g, 56.1 mmol), methylamine hydrochloride (18.9g, 280mmol), *N*-methylmorpholine (34ml, 308mmol), 4-dimethylaminopyridine (685mg, 5.6mmol) and EDC (11.8g, 62mmol) in anhydrous dimethylformamide (300ml) was stirred at RT overnight. The mixture was poured into 10%w/v citric acid (750ml) and extracted with ether (3x500ml). The combined extracts were then washed with 8% sodium bicarbonate (2x500ml) and brine (500ml), dried (MgSO₄) and evaporated *in vacuo* to provide the title compound (12.3g, 83%)
TLC R_{f} 0.47 (50 % hexane-EtOAc)
Similarly prepared was:

### Intermediate 36 (S)-N_{d}-(1,1-Dimethylethoxy)carbonyl-Nₐ-(benzyloxy)carbonylornithine N-methyl amide

From (*S*)-*N*_{*d*}-(1,1 -dimethylethoxy)carbonyl-*N*_{*a*}-(benzyloxy)carbonylomithine (11.5g, 31.4mmol), as a white solid (11.0g, 94%).
TLC R_{f} 0.65 (3% MeOH-CH₂Cl₂)

### Intermediate 37 (R)-N-(1,1-Dimethylethoxy)carbonyl-(S-methyl)penicillamine N-methyl amide

A solution of iodomethane (0.59ml, 9.55mmol) in 3 methanol (5ml) was added dropwise at 0°C to a stirred solution of Intermediate 35 (500mg, 1.51mmol) in a mixture of 2M sodium hydroxide (5ml) and methanol (15ml). The mixture was stirred at RT overnight, then the methanol removed *in vacuo,* the residue diluted with water (50ml) and extracted with ether (3x50ml). The combined extracts were washed with brine, dried (MgSO₄) and evaporated *in vacuo* to provide the title compound as a colourless oil (540mg, 99%).
TLC R_{f} 0.47 (50%hexane-EtOAc)

### Intermediate 38 (S)-b-(2-Pyridyl)alanine N-methyl amide hydrochloride

A solution of Intermediate 33 (1.4g, 5.01 mmol) in a mixture of dioxane (25ml) and 2M hydrochloric acid (25ml) was stirred at RT overnight. The solvent was evaporated to dryness *in vacuo* and freeze dried overnight to provide the title compound as a white solid (1.27g, 100%).
TLC R_{f} 0,13 (1% NEt₃-10% MeOH-CH₂Cl₂)
Similarly prepared was:

### Intermediate 39 (S)-b-(4-Thiazolyl)alanine N-methyl amide hydrochloride

From Intermediate 34 (1.0g, 3.5mmol), as a white solid (730mg, 94%).
TLC R_{f} 0.21 (1% NEt₃-10% MeOH-CH₂Cl₂)

### Intermediate 40 (R)-(S-Methyl)penicillamine N-methyl amide trifluoroacetate

A solution of Intermediate 37 (200mg, 0.72mmol) in dichloromethane (4ml) containing trifluoroacetic acid (2ml) was stirred at RT overnight. The solvent was removed *in vacuo* and any excess trifluoroacetic acid removed by azeotropic distillation with heptane (3x20ml) to provide the title compound (196mg, 94%) as a colourless foam.
TLC R_{f} 0.32 (1% NEt₃-10% MeOH-CH₂Cl₂)

### Intermediate 41 (S)-N_{d}-(1,1-Dimethylethoxy)carbonylomithineN-methylamide

Intermediate 36 (11g, 29mmol) was hydrogenated at RT and atmospheric pressure over 10% palladium on carbon (1g) in ethanol overnight. The catalyst was removed by filtration through hyflo and the filtrate evaporated *in vacuo* to provide the title compound (2.6g, 36%) as a colourless oil.
TLC R_{f} 0.37 (5% MeOH-CH₂Cl₂)

### Intermediate 42 N-(Phenyl methoxy)carbonyl-(S-methyl)-L-cysteinyl-L phenylalanine N-methyl amide

EDC (10.5g, 55mmol) was added to a stirred solution of *L*-phenylalanine *N*-methyl amide (8.9g, 50mmol), Intermediate 26 (13.8g, 50mmol) and *N*-hydroxybenzotriazole (8.1g, 60mmol) in dry tetrahydrofuran (100ml). The mixture was stirred at RT overnight. The mixture was treated with 1M hydrochloric acid (300ml) then extracted with ethyl acetate (4x200ml). The combined organic extracts were washed with 8% sodium bicarbonate (2x200ml), water (200ml) and brine (200ml), dried (MgSO₄) and evaporating *in vacuo* to provide the product as a white solid (16.5g, 75%).
TLC R_{f} 0.47 (10% MeOH-CHCl₃)
Similarly prepared was:

### Intermediate 43 N-(1,1-Dimethylethoxy)carbonyl-(S)-propylglycinyl-L-phenylalanine N-methyl amide

From Intermediate 28 (5g, 23mmol) and *L*-phenylalanine *N*-methyl amide (4.1g, 23mmol), as a white solid (7.72g, 89%).
TLC R_{f} 0.48 (10% MeOH-CH₂Cl₂)

### Intermediate 44 N-(1,1-Dimethylethoxy)carbonyl-L-leucyl-L-tert-leucine N-methyl amide

From *N*-(1,1-dimethylethoxy)carbonyl-*L*-leucine (13.07g, 56mmol) and *L*-*tert*-leucine *N*-methyl amide (8.11g, 56mmol), as a white solid (15.77g, 79%).
TLC R_{f} 0.25 g% MeOH-CH₂Cl₂)

### Intermediate 45 N-(1,1-Dimethylethoxy)carbonyl-(S)-(S-methyl)cysteinyl-L-tert-leucine N-methyl amide

From Intermediate 27 (6.64g, 28mmol) and *L*-*tert*-leucine *N*-methyl amide (4,07g, 28mmol), as a white solid (4.26g, 42%).
TLC R_{f} 0.45 (10% MeOH-CH₂Cl₂)

### Intermediate 46 N-(Phenylmethoxy)carbonyl-L-valinyl-N_{d}-(1,1-dimethylethoxy) carbonyl-L-ornithine N-methyl amide

From Intermediate 41 (2.6g, 10.6mmol) and *N*-(Phenylmethoxy)carbonyl-*L*-valine (2.82g, 11.2mmol), as a white solid (3.0g, 61%).
TLC R_{f} 0.32 (5 % MeOH-CH₂Cl₂)

### Intermediate 47 (S-Methyl)-L-cysteinyl-L-phenylalanine N-methyl amide

A solution of Intermediate 42 (2.0g, 4.65mmol) in dichloromethane (10ml) was treated with 25% hydrobromic acid in acetic acid (18.6ml) and the mixture stirred at RT for lh. Water (10ml) was added and the mixture washed with dichloromethane (3x15ml). The aqueous phase was then basified to pH14 with 5M sodium hydroxide then extracted with dichloromethane (4x30ml). The combined organic extracts were then washed with brine (30ml), dried (MgSO₄) and evaporated *in vacuo* to provide the product as a white solid (1.22g, 88%).
TLC R_{f} 0.31 (10% MeOH-CHCl₃)

### Intermediate 48 L-Valinyl-N_{d}-(1,1-dimethylethoxy)carbonyl-L-ornithine N-methylamide

Intermediate 46 (3.0g, 6.5mmol) was hydrogenated at RT and atmospheric pressure over 10% palladium on carbon (300mg) in ethanol (100ml) overnight. The catalyst was removed by filtration through hyflo and the filtrate evaporated *in vacuo* to provide the title compound (2.2g, 99%) as a white solid.
TLC R_{f} 0.26 (10% MeOH-CH₂Cl₂)

### Intermediate 49 (S)-Propylglycinyl-L-phenylalanine N-methyl amide

A solution of Intermediate 43 (5.36g, 14.2mmol) in a mixture of dioxane (250ml) and 2M hydrochloric acid (250ml) was stirred at RT overnight. The solvent was evaporated to dryness *in vacuo* to provide a white solid. The residue was dissolved in water (200ml) washed with dichloromethane (3x100ml) then basified to pH 10 with 2M sodium hydroxide and extracted with dichloromethane (4x100ml). The combined extracts were washed with brine (100ml), dried MgSO₄) and evaporated *in vacuo* to provide the title compound as a white solid (1.45g, 37%).
TLC R_{f} 0.29 (10% MeOH-CH₂Cl₂)
Similarly prepared were:

### Intermediate 50 (S)-(S-Methyl)cysteinyl-L-tert-leucine N-methyl amide

From Intermediate 45, as a white solid (3.5g, 97%).
TLC R_{f} 0.45 (10% MeOH-CH₂Cl₂)

### Intermediate 51 L-Leucyl-L-tert-leucine N-methyl amide

From Intermediate 44, as a white solid (11.2g, 99%).
TLC R_{f} 0.57 (10% MeOH-CH₂Cl₂)

### Intermediate 52 (R)-2-Bromo-5-phthalimidopentanoic acid

A solution of *D*-ornithine hydrochloride (35g, 0.208mol) in water (350ml) was treated with copper (II) sulphate (16.6g, 0,104mol). 5M Potassium hydroxide *(ca.* 40ml) was added to pH 3 then *N*-carboethoxyphthalimide (45.5g, 0.208mol) was added and the pH maintained at 9-10 by the addition of 5M potassium hydroxide (*ca*.55ml). After 2h, 48% hydrobromic acid was added to pH 0.4 *(ca.* 77ml) and any resulting precipitate removed by filtration. The filtrate was cooled to <5°C, then treated with further hydrobromic acid (152ml) and potassium bromide (59g, 0.5mol). The mixture was then treated dropwise over 45 min with a solution of sodium nitrite (28.6g, 0.41mol) in water (275ml) whilst maintaining a temperature of < 5°C. The mixture was then stirred at <5°C overnight. The resulting precipitate was then removed by filtration, dissolved in ethyl acetate (400ml) and the solution washed with water (2x200ml) and brine (200ml), dried MgSO₄) and evaporated *in vacuo* to provide the title compound (40.6g, 47%) as a cream solid.
TLC R_{f} 0.80 (10% MeOH-CH₂Cl₂)
Similarly prepared was:

### Intermediate 53 (S)-2-Bromo-5-phthalimidopentanoic acid

From L-ornithine hydrochloride (20g, 0.118mol), as a cream solid (22g, 57%).
TLC R_{f} 0.80 (10% MeOH-CH₂Cl₂)

### Intermediate 54 (S)-2-Acetylmercapto-5-phthalimidopentanoic acid

Was prepared by the previously described procedure from Intermediate 52 (39.9g, 0.11mol), as a pale orange oil (35.6g, 99%).
TLC R_{f} 0.24 (50% Heptane-EtOAc)

### Intermediate 55 (R)-2-Acetylmercapto-5-phthalimidopentanoic acid

Was prepared by the previously described procedure from Intermediate 53 (20g, 56mmol), as a pale orange oil (17.7g, 99%).
TLC R_{f} 0.24 (50% Heptane-EtOAc)

### Intermediate 56 (RS)-2-(Acetylmercapto)-5-phthalimidopentanoyl-L-leucine 1,1-dimethylethyl ester

EDC (3.64g, 19mmol) was added to a stirred mixture of L-leucine 1,1-dimethylethyl ester (3.93g, 17.6mmol), *N*-hydroxybenzotriazole (2.62g, 19.4mmol), triethylamine (2.51ml, 18mmol) and Intermediate 17 (5.94g, 18.5mmol) in dry tetrahydrofuran (200ml). The mixture was stirred overnight then the solvent removed *in vacuo* and the residue partitioned between water (100ml) and ethyl acetate (100ml). The aqueous portion was then extracted with ethyl acetate (2x50ml), the combined extracts washed with water (2x100ml) and brine (100ml), dried (MgSO₄) and evaporated *in vacuo* to a colourless oil. Purification by column chromatography eluting with hexane/ethyl acetate (2:1) provided the title compound (6.6g, 77%) as a white solid, a 1:1 mixture of diastereoisomers.
TLC R_{f} 0.42 (EtOAc/Hexane (1:1))

### Intermediate 57 (RS)-2-(Acetylmercapto)-5-phthalimidopentanoyl-L-leucine

Trifluoroacetic acid (9.0ml, 115mmol) was added to a stirred solution of Intermediate 56 (3.0g, 6.1mmol) in dry dichloromethane (40ml) and the mixture stirred at RT overnight. The mixture was concentrated *in vacuo* and the excess trifluoroacetic acid removed by azeotroping with heptane to provide the title compound (2.48g, 94%) as a colourless foam, a 1:1 mixture of diastereoisomers.
TLC R, 0.42 (EtOAc/Hexane (3:2))

### Intermediate 58 (RS)-2-(Acetylmercapto)-4-succinimidobutanoic acid

Was prepared by the procedure previously described for Intermediate 17.
TLC R_{f} 0.38 (HOAc/EtOAc/Hexane (0.1:1:1))

### Intermediate 59 (S-Methyl)-L-cysteinyl-L-tryptophan N-methyl amide

Was prepared by the procedure previously described for Intermediate 47.
TLC R_{f} 0.45 (EtOAc/Hexane (1:1))

### Example 1 (RS)-N-[2,3-Bis-acetylmercaptopropanoyl]-L-leucyl-L-phenylalanine N methyl amide

EDC (198 mg) was added to a solution of Intermediate 12 (209 mg), *L*-leucyl-*L*-phenylalanine *N*-methyl amide (274 mg), and *N*-hydroxybenzotriazole hydrate (153 mg) in dry THF (10 ml) at 0°C, and the mixture was stirred at that temperature until TLC analysis (5% MeOH-CH₂Cl₂) indicated a complete consumption of starting materials (72 h). The solvent was removed by evaporation and the residue was partitioned between 1N hydrochloric acid (35 ml) and ethyl acetate (50 ml). The organic layer was separated, washed with aqueous sodium bicarbonate solution (2 x 200 ml) and brine (2 x 20 ml), then dried (MgSO₄) and evaporated *in vacuo to* give the crude product. Purification by flash column chromatography (eluting with 2-5 % methanol-dichloromethane) gave the title compound as a colourless solid (264 mg).
TLC R_{f} 0.25 (2 % MeOH-CH₂Cl₂)
Similarly prepared were:

### Example 2 (RS)-N-[2-Acetylmercapto-3-methoxycarbonylpropanoyl]-L-leucyl-L-phenylalanine N-methyl amide

From Intermediate 4 (0.26 g), as a colourless solid (0.33 g).
C₂₃H₃₃N₃O₆S [479.6]; [MH⁺ =480]

### Example 3 (RS)-N-[2-Acetylmercapto-4-methoxycarbonylbutanoyl]-L-leucyl-L-phenylalanine N-methyl amide

From Intermediate 5 (0.40 g), as a colourless solid (0.67 g).
C₂₄H₃₅N₃O₆S [493.6]; [MH+ = 494]

### Example 4 (RS)-N-[2-Acetylmercapto-5-methoxycarbonylpentanoyl]-L-leucyl-L-phenylalanine N-methyl amide

From Intermediate 6 (0.9 g), as a colourless solid (1.1 g).
C₂₅H₃₇N₃O₆S [507.6]; [MH⁺ =508]

### Example 5 (RS)-N-[2-Acetylmercapto-6-methoxycarbonylhexanoyl]-L-leucyl-L-phenylalanine N-methyl amide

From Intermediate 7 (0.95 g), as a colourless solid (1.0 g).
C₂₆H₃₉N₃O₆5 [521.6]; [MH⁺ = 522]

### Example 6 (RS)-N-(2-Acetylmercapto-5-phthalimidopentanoyl]-L-leucyl-L-tryptophan N-methyl amide

From Intermediate 17 (730mg) and *L*-leucyl-*L*-tryptophan *N*-methyl amide (700mg), as a pale yellow foam (1.0g, 74%)
C₃₃H₃₉N₅O₆S [633.7]; [MH⁺ =634]

### Example 7 (RS)-N-[2-Acetylmercapto-6-methoxycarbonylhexanoyl]-L-leucyl-L-tryptophan N-methyl amide

From Intermediate 7 (375mg) and *L*-leucyl-*L*-tryptophan *N*-methyl amide (500mg), as a pale yellow foam (440mg, 52%)
C₂₈H₄₀N₄O₆S [560.7]; [MH⁺=561]

### Example 8 (S)-N-[2-(Acetylmercapto)-5-phthalimido]pentanoyl-L-leucyl-(S)-tert-leucine N-methyl amide

From Intermediate 54 (13.8g, 43mmol) and Intermediate 51 (11.2g, 44mmol), as a white solid (11.6g, 48%).
C₂₈H₄₀N₄O₆S [560.7]; [MH⁺=561]

### Example 9 (S)-N-[2-(Acetylmercapto)-5-phtalimido]pentanoyl-L-leucyl-L-phenylalanine N-methyl amide

From Intermediate 54 (10g, 31 mmol) and *L*-leucyl-*L*-phenylalanine *N*-methyl amide (9.0g, 31mmol), as a white solid (10.5g, 57%).
C₃₁H₃₈N₄O₆S [594.7]; [MH⁺ = 595]

### Example 10 (R)-N-[2-(Acetylmercapto)-5-phthalimido]pentanoyl-L-leucyl-L-phenylalanine N-methyl amide

From Intermediate 55 (1.0g, 3.1 mmol) and *L*-leucyl-*L*-phenylalanine *N*-methyl amide (900mg, 3.1mmol), as a white solid (885mg, 48%).
C₃₁H₃₈N₄O₆S [594.7]; [MH⁺ = 595]

### Example 11 (S)-N-[2-(Acetylmercapto)-5-phthalimido]pentanoyl-L-leucyl-L-tryptophan N-methyl amide

From Intermediate 54 (411mg, 1.28mmol) and *L*-leucyl-*L*-tryptophan *N*-methyl amide (423mg, 1.28mmol), as a white solid (330mg, 41%).
C₃₃H₃₉N₅O₆S [633.7]; [MH⁺ = 634]

### Example 12 (RS)-N-[2-(Acetylmercapto)-5-phthalimido]pentanoyl-L-valinyl-N_{d}-(1,1-dimethylethoxy)carbonyl-L-ornithine N-methyl amide

From Intermediate 48 (508mg, 1.47mmol) and Intermediate 17 (491mg, 1.53mmol), as a white solid (687mg, 72%).
C₃₁H₄₅N₅O₈S [647.8] ; [MH ⁺ = 648]

### Example 13 (RS)-N-[2-(Acetylmercapto)-5-phthalimido]pentanoyl-L-valinyl-L-ornithine N-methyl amide trifluoroacetate

A solution of Example 12 (585mg, 0.90mmol) in dichloromethane (20ml) containing trifluoroacetic acid (2ml) was stirred at RT overnight. The solvent was removed *in vacuo* and any excess trifluoroacetic acid removed by azeotropic distillation with heptane (3x20ml) to provide the title compound (596mg, 99%) as a off white solid.
C₂₆H₃₈N₅O₆S [548.7]; [MH⁺ = 549]

### Example 14 (RS)-N-(2-Acetylmercapto-5-phthalimidopentanoyl]-L-leucyl-L-phenylalanine N-methyl amide

A solution of potassium thiolacetate (98 mg) in methanol (2 ml) was added to a suspension of Intermediate 18 (0.5 g) in methanol (10 ml) and the mixture was stirred at RT for 30 min, then at reflux for 6h. The solvent was then removed under reduced pressure and the residue was partitioned between water (50 ml) and dichloromethane (150 ml). The layers were separated and the organic layer was dried over sodium sulfate, filtered, and evaporated to give the crude product. Purification by flash column chromatography (eluting with 50% ethyl acetate-dichloromethane) gave the title compound as a near colourless solid (0.42g).
TLC R_{f} 0.20 (50% EtOAc-CH₂Cl₂)

### Example 15 (RS)-N-[2-(Acetylmercapto)-5-phthalimido]pentanoyl-L-leucyl-L-[β-(4-thiazolyl)]alanine N-methyl amide

EDC (107mg, 0.56mmol) was added to a stirred solution of Intermediate 57 (222mg,0.51 mmol), *N*-hydroxybenzotriazole 76mg, 0.56mmol), triethylamine (75 ml, 0.53mmol) and Intermediate 39 (113mg, 0.5lmmol) in dry tetrahydrofuran (30ml). The mixture was stirred at RT overnight then the solvent removed *in vacuo* and the residue partitioned between water (20ml) and ethyl acetate (20ml). The aqueous portion was then extracted with ethyl acetate (2x20ml), the combined extracts washed with water (2x50ml) and brine (50ml), dried (MgSO₄) and evaporated *in vacuo* to a pale yellow oil.

Purification by column chromatography eluting with dichloromethane/methanol (98:2) provided the title compound (170mg, 55%) as a white solid.
C₂₁H₃₅N₅O₆S₂ [601.7]; [MH⁺ = 602]
Similarly prepared were:

### Example 16 (RS)-N-[2-(Acetylmercapto)-5-phthalimido]pentanoyl-L-leucyl-L-[β-(2-pyridyl)]alanine N-methyl amide

From Intermediate 57 and Intermediate 38, as a white solid (277mg, 67%).
C₃₀H₃₇N₅O₆S [595.7]; [MH⁺ = 596]

### Example 17 (RS)-N-[2-(Acetylmercapto)-5-phthalimido]pentanoyl-L-leucyl-(R)-(S-methyl)-penicillamine N-methyl amide

From Intermediate 57 and Intermediate 40, as a white solid (400mg, 35%).
C₂₈H₄₀N₄O₆S₂ [592.8]; [MH⁺ = 593]

### Example 18 (RS)-N-[2-(Acetylmercapto)-5-phthalimido]pentanoyl-L-leucyl-(S)-tert-leucine N-methyl amide

From Intermediate 57 and *tert*-leucine *N*-methyl amide hydrochloride, as a white solid (120mg, 25%).
C₂₈H₄₀N₄O₆S [560.7]; [MH⁺=561]

### Example 19 (RS)-N-[2-Mercapto-5-(succinimido)pentanoyl]-L-leucyl-L-phenylalanine N-methyl amide

0.4M Sodium hydroxide (0.82ml) was added to a stirred solution of Intermediate 24 (600 mg, 0.91 mmol) and 2-mercaptoethanol (0.23ml, 3.28mmol) in methanol at 0°C. After 15min acetic acid (0.5ml) was added and the solvents evaporated *in vacuo* to provide a yellow oil. Ether (20ml) was added and the resulting precipitate removed by filtration to provide the crude thiol. Purification by flash column chromatography (eluting with 5% methanol in dichloromethane) provided the title compound (147 mg, 32%) as a white solid.

Similarly prepared was :

### Example 20 (RS)-N-[2-Mercapto-5-(4-pyridylcarbonyl)aminopentanoyl]-L-leucyl-L phenylalanine N-methyl amide

From Intermediate 25 (330mg, 0.48mmol), as a white solid (115mg, 22%).
C₂₇H₃₇N₅O₄S [527.7]; [MH⁺ = 528]

### Example 21 (RS)-N-[2-Mercapto-5-phthalimido]pentanoyl-L-leucyl-L-[β-(4- thiazolyl)]alanine N-methyl amide

Concentrated ammonium hydroxide (0.5ml) was added to a solution of Example 15 (110mg, 0.18mmol) in methanol (10ml) at 0°C and the mixture was stirred at that temperature for 3 h. The mixture was diluted with water (10 ml), acidified with 2N aqueous hydrochloric acid and extracted with dichloromethane (3x20ml). The combined extracts were dried (Na₂SO₄) filtered and evaporated to give the crude product. Purification by flash column chromatography (eluting with 2% methanol-dichloromethane) gave the title compound as a colourless solid (71mg, 71 %).
C₂₆H₃₃N₅O₅S₂ [559.7]; [MH⁺=560]
Similarly prepared were:

### Example 22 (RS)-N-[2-Mercapto-5-phthalimido]pentanoyl-L-leucyl-L-[β-(2-pyidyl)]alanine N-methyl amide

From Example 16, as a white solid (75mg, 80%).
C₂₈H₃₅N₅O₅S [553.7]; [MH⁺ = 554]

### Example 23 (RS)-N-[2-Mercapto-5-phthalimido]pentanoyl-L-leucyl-(R)-(S-methyl)-penicillamine N-methyl amide

From Example 17, as a white solid (160mg, 70%).
C₂₆H₃₈N₄O₅S₂ [550.8]; [MH⁺ = 551]

### Example 24 (S)-N-[2-Mercapto-5-phthalimido]pentanoyl-L-leucyl-(S)-tert-leucine N-methyl amide

From Example 8, as a white solid (8.12g, 80%).
C₂₆H₃₈N₄O₅S [518.7]; [MH⁺ = 519]

### Example 25 (S)-N-[2-Mercapto-5-phthalimido]pentanoyl-L-leucyl-L-phenylalanine N-methyl amide

From Example 9, as a white solid (2.7g, 67%).
C₂₉H₃₆N₄O₅S [552.7]; [MH⁺ = 553]

### Example 26 (RS)-N-[2,3-Dimercaptopropanoyl]-L-leucyl-L-phenylalanine N-methyl amide

From Example 1, as a colourless solid (76 mg).
C₁₉H₂₉N₃O₃S₂ [411.5]; [MH⁺ = 412]

### Example 27 (RS)-N-[2-Mercapto-5-methoxycarbonylpentanoyl]-L-leucyl-L-phenylalanine N-methyl amide

From Example 4 (0.32 g), as a colourless solid (0.15 g).
TLC R_{f} 0.29 (5 % MeOH-CH₂Cl₂)

### Example 28 (RS)-N-[2-Mercapto-6-methoxycarbonylhexanoyl]-L-leucyl-L-phenylalanine N-methyl amide

From Example 5 (0.31 g), as a colourless solid (0.22 g).
TLC R_{f} 0.30 (5% MeOH-CH₂Cl₂)

### Example 29 (RS)-N-[2-Mercapto-5-phthalimidopentanoyl]-L-leucyl-L-phenylalanine N-methyl amide

From Example 14 (0.2 g), a a colourless solid (0.18 g).
TLC R_{f} 0.41 (5% MeOH-CH₂Cl₂)

### Example 30 (RS)-N-(2-Mercapto-3-phthalimidopentanoyl]-L-leucyl-L-tryptophanN-methyl amide

From Example 6 (250mg), as a pale yellow foam (222mg, 96%).
C₃₁H₃₇N₅O₅S [591.7]; [MH⁺ = 592]

### Example 31 (RS)-N-[2-Mercapto-6-methoxycarbonylhexanoyl]-L-leucyl-L-tryptophan N-methyl amide

From Example 7 (330mg), as a colourless foam (300mg, 98%).
C₂₆H₃₁N₄O₅S [518.7]; [MH⁺ = 519]

### Example 32 (S)-N-[2-Mercapto-5-phthalimidopentanoyl]-L-(S-methyl)cysteinyl-L-tryptophan N-methyl amide

Was prepared by the procedure described previously for Examples 15 and 21, from Intermediate 54 and Intermediate 59.
C₂₉H₃₂N₅O₅S₂ [595.7]; [MH⁺ = 596]

### Example 33 (RS)-N-[2-Mercapto-4-succinimidobutanoyl]-L-leucyl-L-phenylalanine N-methyl amide

Was prepared by the procedure described previously for Examples 15 and 21, from Intermediate 58 and *L*-leucyl-*L*-phenylalanine *N*-methyl amide.
C₂₄H₃₄N₄O₅S [490.6]; [MH⁺ = 491]

### Example 34 (RS)-N-[2-Mercapto-4-succinimidobutanoyl]-(S)-propylglycinyl-L-phenylalanine N-methyl amide

Was prepared by the procedure described previously for Examples 15 and 21, from Intermediate 58 and Intermediate 49.
C₂₃H₃₁N₄O₅S [475.6]; [MH⁺ = 476]

### Example 35 (RS) -N-[2-Mercapto-4-succinimidobutanoyl]-L-(S-methyl)cysteinyl-L-tert-leucine N-methyl amide

Was prepared by the procedure described previously for Examples 15 and 21, from Intermediate 58 and Intermediate 50.
C₁₉H₃₂N₄O₅S₂ [460.6]; [MH⁺ = 461]

### Example 36 (RS)-N-[2-Mercapto-4-succinimidobutanoyl]-L-leucyl-L-tert-leucine N-methyl amide

Was prepared by the procedure described previously for Examples 15 and 21, from Intermediates 58 and 51.
C₂₁H₃₆N₄O₅S [456.6]; [MH⁺ = 457]

### Example 37 (R)-N-[2-Acetylmercapto-5-phthalimidopentanoyl]-L-leucyl-L-tryptophan N-methyl amide

Was prepared by separation of the 1:1 mixture of diastereoisomers present in Example 6 by flash column chromatography.
C₃₁H₃₇N₅O₅S₂ [591.7]; [MH⁺ = 592]

### Example 38 (RS)-N-[2-Mercapto-6-methoxycarbonylhexanoyl]-L-leucyl-L-phenylalanine N-methyl amide

Was prepared by hydrolysis of Example 5.
C₂₄H₃₈N₄O₄S [478.6]; [MH⁺ = 479]

## Claims

1. A compound of general formula (I): 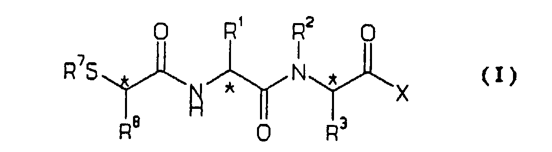 wherein:
R¹ is C₁₋₆ alkyl, C₂₋₆ alkenyl, -C₁₋₆ alkyl-aryl, aryl, -C₁₋₆ alkyl-heteroaryl, heteroaryl or -C₁₋₆ alkyl-AR⁹ where A represents O,NR⁹ or S(O)ₘ where m=0-2, and R⁹ is H, C₁₋₄ alkyl, aryl, heteroaryl, -C₁₋₆ alkyl-aryl or -C₁₋₄ alkyl-heteroaryl; if A=NR⁹ the groups R⁹ may be the same or different;
R² is H or C₁₋₆ alkyl;
R³ is [Alk]ₙR⁶ where Alk is C₁₋₆ alkyl or C₂₋₆ alkenyl and n is zero or 1;
X is NR⁴R⁵ where either R⁴ is hydrogen or C₁₋₆ alkyl optionally substituted by amino (NH₂), aryl, arylamino, protected amino, di(C₁₋₆ alkyl)amino, mono(C₁₋₆ alkyl)amino, CO₂H, protected carboxyl, carbamoyl, mono(C₁₋₆ alkyl)carbamoyl or di(C₁₋₆ alkyl)carbamoyl, and R⁵ is hydrogen or C₁₋₆ alkyl; or NR⁴R⁵ forms a ring such as pyrrolidino, piperidino or morpholino;
R⁷ is hydrogen or R¹⁰CO where R¹⁰ is C₁₋₄ alkyl, -C₁₋₄ alkyl-aryl, -C₁₋₄ alkyl-heteroaryl, cyclo(C₃₋₆)alkyl, -C₁₋₄ alkyl-cyclo(C₃₋₆)alkyl, C₂₋₆ alkenyl, -C₂₋₆ alkenylaryl, aryl or heteroaryl;
R⁸ is aryl (substituted with R¹¹), heteroaryl (optionally substituted with R¹¹), C₁₋₄ alkyl-R¹¹, -C₁₋₄ alkyl-aryl (substituted with R¹¹), -C₁₋₄ alkyl-heteroaryl (optionally substituted with R¹¹), cyclo(C₃₋₆)alkyl (optionally substituted with R¹¹), cyclo(C₃₋ 6)alkenyl (optionally substituted with R¹¹), -C₁₋₄ alkyl-cyclo(C₃₋₆)alkyl (optionally substituted with R¹¹), or any of the three groups 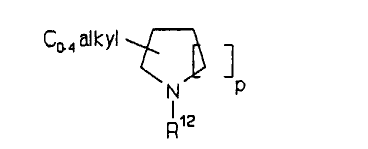 or 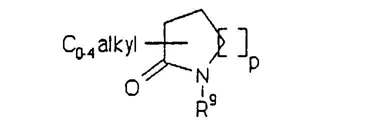 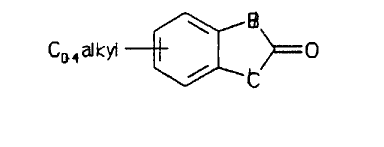 where p is 1 or 2 and B and C are independently selected from 0, S, C(R⁹)₂ and NR⁹;
R⁶ is AR⁹, cycle(C₃₋₆)alkyl, cyclo(C₃₋₆)alkenyl, C₁₋₆ alkyl, -C₁₋₆ alkoxy-aryl, benzyloxyaryl, aryl, heteroaryl, -C₁₋₃ alkyl-heteroaryl, -C₁₋₃ alkyl-aryl, -C₁₋₆ alkyl-COOR⁹, -C₁₋₆ alkyl-NHR, CONHR, NHCO₂R, NHSO₂R or NHCOR, R being defined as for R¹⁰;
R¹¹ is SO₂R¹³, SR⁷, SR⁹, COR¹³, N(R⁹)2, NR⁹R¹², OR⁹, succinimido or phthalimido;
R¹² is H or COR⁹ CO₂R⁹ (where R⁹ is not H), CONHR⁹ or SO₂R⁹ (where R⁹ is not H); and
R¹³ is OH, OC₁₋₄alkyl, O-C₁₋₄ alkyl-aryl, N(R⁹)₂ (in which the R⁹s are the same or different), C₁₋₄ alkyl, aryl, heteroaryl, -C₁₋₄ alkyl-aryl or -C₁₋₄ alkyl-heteroaryl;
the compound being in the form of a non-salt, salt, solvate or hydrate.

2. A compound of claim 1, wherein R¹ is C₁₋₆ alkyl or C₁₋₆ alkyl-AR⁹ where A is S(O)ₘ, NR⁹ or O and m=0, 1 or 2, and R⁹ is H, C₁₋₄ alkyl or aryl.

3. A compound of claim 1 or claim 2, wherein R³ is [Alk]ₘR⁶ where n=0 or 1, Alk is C₁₋₆ alkyl and R⁶ is C₁₋₆ alkyl, -C₁₋₃ alkyl-aryl, -C₁₋₃ alkyl-heteroaryl or AR⁹.

4. A compound of any preceding claim, wherein R⁴ is H.

5. A compound of any preceding claim, wherein X is pyrrolidino, piperidino or morpholino.

6. A compound of any preceding claim, wherein R⁷ is H or (C₁₋₆ alkyl)carbonyl.

7. A compound of any preceding claim, wherein R⁸ is C₁₋₄ alkyl-R¹¹ or cyclo(C₃₋₆)alkyl-R¹¹, and R¹¹ is COR¹³, NR⁹R¹², N(R⁹)₂, succinimido or phthalimido, R¹² is COR⁹, CO₂R⁹ (provided R⁹ is not H) or SO₂R⁹ (provided R⁹ is not H), and R¹³ is OH, OC₁₋₄ alkyl or N(R⁹)₂.

8. A compound of any preceding claim, wherein R⁵ is H or C₁₋₆ alkyl.

9. A compound of claim 8, wherein:
R¹ is alkyl, alkenyl, alkylaryl, aryl or alkyl-AR⁹ and R⁹ is alkyl, aryl or heteroaryl;
R⁷ is H or R¹⁰CO where R¹⁰ is alkyl, alkylaryl, cycloalkyl, cycloalkylalkyl, alkenyl or alkenylaryl;
R⁸ is optionally-substituted aryl, heteroaryl, alkylaryl, cycloalkyl, cycloalkenyl or alkylcycloalkyl, alkyl-R¹¹ or any of the said three groups;
R⁶ is cycloalkyl, cycloalkenyl, alkyl, benzyl, alkoxybenzyl, benzyloxylbenzyl or 3-indolylmethyl;
R¹¹ is SR⁷, SR⁹, COR¹³, N(R⁹)₂, NR⁹R¹², OR⁹, succinimido or phthalimido; and
R¹³ is OH, Oalkyl, Oalkylaryl, N(R⁹)₂, alkyl, aryl or alkylaryl.

10. A compound of claim 1, selected from
*N*-[2,3-*bis*-Acetyl mercaptopropanoyl]-*L*-leucyl-*L*-phenylalanine *N*-methylamide
*N*-[2-Acetylmercapto-3-methoxycarbonylpropanoyl]-*L*-leucyl-*L*-phenylalanine *N*-methylamide
*N*-[2-Acetylmercapto-4-methoxycarbonylbutanoyl]-*L*-leucyl-*L*-phenylalanine *N*-methylamide
*N*-[2-Acetylmercapto-5-methoxycarbonylpentanoyl]-*L*-leucyl-*L*-phenylalanine *N*-methylamide
*N*-[2-Acetylmercapto-6-methoxycarbonylhexanoyl]-*L*-leucyl-*L*-phenylalanine *N*-methylamide
*N*-[2-Acetylmercapto-4-phthalimidobutanoyl]-*L*-leucyl-*L*-phenylalanine *N*-methylamide
*N*-[2-Acetylmercapto-5-phthalimidopentanoyl]-*L*-leucyl-*L*-phenylalanine *N*-methylamide
*N*-[2-Acetylmercapto-6-phthalimidohexanoyl]-*L*-leucyl-*L*-phenylalanine *N*-methylamide
*N*-[2,3-bis-Mercaptopropanoyl]-*L*-leucyl-*L*-phenylalanine *N*-methylamide
*N*-[2-Mercapto-3-methoxycarbonylpropanoyl]-*L*-leucyl-*L*-phenylalanine *N*-methylamide
*N*-[2-Mercapto-4-methoxycarbonylbutanoyl]-*L*-leucyl-*L*-phenylalanine *N*-methylamide
*N*-[2-Mercapto-5-methoxycarbonylpentanoyl]-*L*-leucyl-*L*-phenylalanine *N*-methylamide
*N*-[2-Mercapto-6-methoxycarbonylhexanoyl]-*L*-leucyl-*L*-phenylalanine *N*-methylamide
*N*-[2-Mercapto-4-phthalimidobutanoyl]-*L*-leucyl-*L*-phenylalanine *N*-methylamide
*N*-[2-Mercapto-5-phthalimidopentanoyl]-*L*-leucyl-*L*-phenylalanine*N*-methylamideand
*N*-[2-Mercapto-6-phthalimidohexanoyl]-*L*-leucyl-*L*-phenylalanine *N*-methylamide.

11. A compound of claim 1, selected from
*N*-[2-Acetylmercapto-5-methoxycarbonylpentanoyl]-*L*-leucyl-*L*-phenylalanine *N*-methylamide
*N*-[2-Acetylmercapto-6-methoxycarbonylhexanoyl]-*L*-leucyl-*L*-phenylalanine *N*-methylamide
*N*-[2-Acetylmercapto-6-methoxycarbonylhexanoyl]-*L*-valinyl-*L*-phenylalanine *N*-methylamide
*N*-[2-Acetylmercapto-6-methoxycarbonylhexanoyl]-*L*-leucyl-*L*-tryptophan N-methylamide
*N*-[2-Acetylmercapto-5-phthalimidopentanoyl]-*L*-leucyl-*L*-phenylalanine *N*-methylamide
*N*-[2-Acetylmercapto-5-phthalimidopentanoyl]-*L*-valinyl-*L*-phenylalanine *N*-methylamide
*N*-[2-Acetylmercapto-5-phthalimidopentanoyl]-*L*-leucyl-*L*-tryptophan *N*-methylamide
*N*-[2-Acetylmercapto-5-phthalimidopentanoyl]-*L*-leucyl-*L*-[β-(4-thiazolyl)]alanine*N*-methylamide
*N*-[2-Acetylmercapto-5-phthalimidopentanoyl]-*L*-leucyl-*L*-[β-(2-pyridyl)]alanine *N*-methylamide
*N*-[2-Acetylmercapto-5-phthalimidopentanoyl]-*L*-leucyl-5-methyl-*L*-glutamicacid *N*-methylamide
*N*-[2-Acetylmercapto-6-phthalimidohexanoyl]-*L*-leucyl-*L*-phenylalanine *N*-methylamide
*N*-[2-Acetylmercapto-2-(3-phthalimido)phenylacetyl]-*L*-leucyl-*L*-phenylalanine *N*-methylamide
*N*-[2-Mercapto-5-methoxycarbonylpentanoyl]-*L*-leucyl-*L*-phenylalanine *N*-methylamide
*N*-[2-Mercapto-6-methoxycarbonylhexanoyl]-*L*-leucyl-*L*-phenylalanine *N*-methylamide
*N*-[2-Mercapto-6-methoxycarbonylhexanoyl]-*L*-leucyl-*L*-tryptophan *N*-methylamide
*N*-[2-Mercapto-5-phthalimidopentanoyl]-*L*-leucyl-*L*-phenylalanine *N*-methylamide
*N*-[2-Mercapto-5-phthalimidopentanoyl]-*L*-leucyl-*L*-tryptophan *N*-methylamide
*N*-[2-Mercapto-5-phthalimidopentanoyl]-*L*-leucyl-*L*-[β-(4-thiazolyl)]alanine *N*-methylamide
*N*-[2-Mercapto-5-phthalimidopentanoyl]-*L*-leucyl-*L*-[β-(2-pyridyl)]alanine *N*-methylamide
*N*-[2-Mercapto-5-phthalimidopentanoyl]-*L*-leucyl-5-methyl-*L*-glutamic acid *N*-methylamide and
*N*-[2-Mercapto-6-phthalimidohexanoyl]-*L*-leucyl-*L*-phenylalanine *N*-methylamide.

12. A compound of any of claims 1 to 9, wherein R¹ is -CH₂SCH₃.

13. A compound of claim 12, selected from
(*RS*)-*N*-[2-(Acetylmercapto)-5-phthalimidopentanoyl]-(S-methyl)-*L*-cysteinyl-*L*-phenylalanine *N*-methyl amide
(*S*)-*N*-[2-Mercapto-5-phthalimidopentanoyl]-*L*-(*S*-methyl)-cysteinyl-*L*-tryptophan *N*-methyl amide
(*RS*)-*N*-[2-Mercapto-4-succinimidobutanoyl]-*L*-(*S*-methyl)-cysteinyl-*L*-*tert*-leucine *N*-methyl amide
(*RS*)-*N*-[2-Mercapto-4-succinimidobutanoyl]-*L*-(*S*-methyl)-cysteinyl-*L*-tryptophan *N*-methyl amide and
(*RS*)-*N*-[2-Mercapto-4-succinimidobutanoyl]-*L*-(*S*-methyl)-cysteinyl-*L*-phenylalanine *N*-methyl amide.

14. A compound of any of claims 1 to 9, wherein R³ is tert-butyl or C(CH₃)₂S(O)₀₋₂CH₃.

15. A compound of claim 14, selected from
(*S*)-*N*-[2-(Acetylmercapto)-5-phthalimido]pentanoyl-*L*-leucyl-(*S*)-*tert*-leucine *N*-methyl amide
(*RS*)-*N*-[2-(Acetylmercapto)-5-phthalimido]pentanoyl-*L*-leucyl-(*R*)-penicillamine *N*-methyl amide
(*RS*)-*N*-[2-(Acetylmercapto)-5-phthalimido]pentanoyl-*L*-leucyl-(*R*)-(*S*-methyl)-penicillamine *N*-methyl amide
(*RS*)-*N*-[2-(Acetylmercapto)-5-phthalimido]pentanoyl-*L*-leucyl-(*R*)-(2,2-dimethyl-2-methanesulphonyl)alanine *N*-methyl amide
(*RS*)-*N*-[2-(Acetylmercapto)-5-phthalimido]pentanoyl-*L*-leucyl-(*R*)-(2,2-dimethyl-2-methanesulphinyl)alanine *N*-methyl amide
(*RS*)-*N*-[2-(Acetylmercapto)-5-phthalimido]pentanoyl-*L*-leucyl-(*S*)-*tert*-leucine *N*-methyl amide
(*RS*)-*N*-[2-Mercapto-5-phthalimido]pentanoyl-*L*-leucyl-(*R*)-(*S*-methyl)-penicillamine *N*-methyl amide
(*S*)-*N*-[2-Mercapto-5-phthalimido]pentanoyl-*L*-leucyl-(*S*)-*tert*-leucine *N*-methyl amide and
(*RS*)-*N*-[2-Mercapto-4-succinimidobutanoyl]-*L*-leucyl-*L*-*tert*-leucine *N*-methyl amide.

16. A compound of any preceding claim, in the form of a single enantiomer or diastereomer, or a mixture of such isomers.

17. A pharmaceutical composition for use in therapy, comprising a compound of any preceding claim, and a pharmaceutically-acceptable diluent or carrier.

18. Use of a compound of any of claims 1 to 16, for the manufacture of a medicament for the treatment or prevention of a condition associated with matrix metalloproteinases or that is mediated by TNFα.

19. Use according to claim 18, wherein the condition is selected from cancer, inflammation and inflammatory diseases, tissue degeneration, periodontal disease, ophthalmological disease, dermatological disorders, fever, cardiovascular effects, haemorrhage, coagulation and acute phase response, cachexia and anorexia, acute infection, HIV infection, shock states, graft versus host reactions, autoimmune disease, reperfusion injury, meningitis and migraine.

20. Use according to claim 19, wherein the condition is selected from tumour growth, angiogenesis, tumour invasion and spread, metastases, malignant ascites and malignant pleural effusion.

21. Use according to claim 19, wherein the condition is selected from rheumatoid arthritis, osteoarthritis, osteoporosis, asthma, multiple sclerosis, neurodegeneration, Alzheimer's disease, atherosclerosis, stroke, vasculitis, Crohn's disease and ulcerative colitis.

22. Use according to claim 19, wherein the condition is selected from corneal ulceration, retinopathy and surgical wound healing.

23. Use according to claim 19, wherein the condition is selected from psoriasis, atopic dermatitis, chronic ulcers and epidermolysis bullosa.

24. Use according to claim 19, wherein the condition is periodonititis or gingivitis.

## Patentansprüche

1. Verbindung der allgemeinen Formel (I): 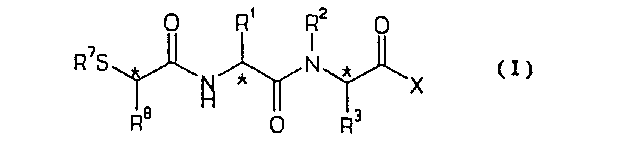 worin:
R¹ C₁₋₆-Alkyl, C₂₋₆-Alkenyl, -C₁₋₆-Alkylaryl, Aryl, -C₁₋₆-Alkylheteroaryl, Heteroaryl oder -C₁₋₆Alkyl-AR⁹ ist, worin A O, NR⁹ oder S(O)ₘ darstellt, wobei m = 0-2 und R⁹ H, C₁₋₄-Alkyl, Aryl, Heteroaryl, -C₁₋₄-Alkylaryl oder -C₁₋₄-Alkyl-heteroaryl ist; wenn A = NR⁹, die Gruppen R⁹ gleich oder verschieden sein können;
R² H oder C₁₋₆-Alkyl ist;
R³ [Alk]ₙR⁶ ist, worin Alk C₁₋₆-Alkyl oder C₂₋₆-Alkenyl ist und n 0 oder 1 ist;
X NR⁴R⁵ ist, wobei entweder R⁴ Wasserstoff oder C₁₋₆-Alkyl, gegebenenfalls substituiert mit Amino (NH₂), Aryl, Arylamino, geschütztem Amino, Di(C₁₋₆-alkyl)amino, Mono(C₁₋₆-alkyl)amino, CO₂H, geschütztem Carboxyl, Carbamoyl, Mono(C₁₋₆-alkyl)carbamoyl oder Di(C₁₋₆alkyl)carbamoyl, ist und R⁵ Wasserstoff oder C₁₋₆-Alkyl ist; oder NR⁴R⁵ einen Ring wie Pyrrolidino, Piperidino oder Morpholino bildet;
R⁷ Wasserstoff oder R¹⁰CO ist, wobei R¹⁰ C₁₋₄-Alkyl, -C₁₋₄-Alkylaryl, -C₁₋₄-Alkylheteroaryl, Cyclo(C₃₋₆)alkyl, -C₁₋₄-Alkylcydo(C₃₋₆)alkyl, C₂₋₆-Alkenyl, -C₂₋₆-Alkenylaryl, Aryl oder Heteroaryl ist;
R⁸ Aryl (substituiert mit R¹¹), Heteroaryl (gegebenenfalls mit R¹¹ substituiert), C₁₋₄-Alkyl-R¹¹, -C₁₋₄-Alkylaryl (substituiert mit R¹¹), -C₁₋₄-Alkyl-heteroaryl (gegebenenfalls mit R¹¹ substituiert), Cyclo(C₃₋₆)alkyl (gegebenenfalls mit R¹¹ substituiert), Cyclo(C₃₋₆)alkenyl (gegebenenfalls mit R¹¹ substituiert), -C₁₋₄-Alkylcyclo(C₃₋₆)alkyl (gegebenenfalls mit R¹¹ substituiert) oder irgendeine der drei Gruppen 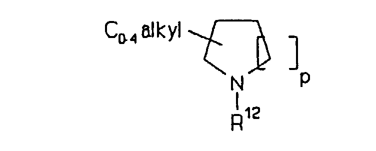 oder 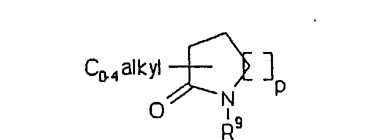 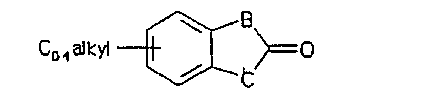 ist, worin p 1 oder 2 ist und B und C unabhängig aus O, S, C(R⁹)₂ und NR⁹ ausgewählt sind;
R⁶ AR⁹, Cyclo(C₃₋₆)alkyl, Cyclo(C₃₋₆)alkenyl, C₁₋₆-Alkyl, -C₁₋₆-Alkoxyaryl, Benzyloxyaryl, Aryl, Heteroaryl, -C₁₋₃-Alkylheteroaryl, -C₁₋₃-Alkylaryl, -C₁₋₆-Alkyl-COOR⁹, -C₁₋₆-Alkyl-NHR, CONHR, NHCO₂R, NHSO₂R oder NHCOR ist, wobei R wie für R¹⁰ definiert ist;
R¹¹ SO₂R¹³, SR⁷, SR⁹, COR¹³, N(R⁹)₂, NR⁹R¹², OR⁹, Succinimido oder Phthalimido ist;
R¹² H oder COR⁹, CO₂R⁹ (worin R⁹ nicht H ist), CONHR⁹ oder SO₂R⁹ (worin R⁹ nicht H ist) ist; und
R¹³ OH, OC₁₋₄-Alkyl, OC₁₋₄-Alkylaryl, N(R⁹)₂ (worin die R⁹ gleich oder verschieden sind), C₁₋₄-Alkyl, Aryl, Heteroaryl, -C₁₋₄-Alkylaryl oder -C₁₋₄-Alkylheteroaryl ist;
wobei die Verbindung in Form eines Nicht-Salzes, Salzes, Solvats oder Hydrats vorliegt.

2. Verbindung nach Anspruch 1, worin R¹ C₁₋₆-Alkyl oder C₁₋₆-Alkyl-AR⁹ ist, wobei A S(O)ₘ, NR⁹ oder O ist und m = 0, 1 oder 2, und R⁹ H, C₁₋₄-Alkyl oder Aryl ist.

3. Verbindung nach Anspruch oder Anspruch 2, worin R³ [Alk]ₘR⁶ ist, wobei n = 0 oder 1, Alk C₁₋₆-Alkyl ist und R⁶ C₁₋₆-Alkyl, -C₁₋₃-Alkylaryl, -C₁₋₃-Alkylheteroaryl oder AR⁹ ist.

4. Verbindung nach irgendeinem der vorhergehenden Ansprüche, worin R⁴ H ist.

5. Verbindung nach irgendeinem der vorhergehenden Ansprüche, worin X Pyrrolidino, Piperidino oder Morpholino ist.

6. Verbindung nach irgendeinem der vorhergehenden Ansprüche, worin R⁷ H oder (C₁₋₆-Alkyl)carbonyl ist.

7. Verbindung nach irgendeinem der vorhergehenden Ansprüche, worin R⁸ C₁₋₄-Alkyl-R¹¹ oder Cyclo(C₃₋₆)alkyl-R¹¹ ist und R¹¹ COR¹³, NR⁹R¹², N(R⁹)₂, Succinimido oder Phthalimido ist, R¹² COR⁹, CO₂R⁹ (vorausgesetzt, daß R⁹ nicht H ist) oder SO₂R⁹ (vorausgesetzt, daß R⁹ nicht H ist) ist, und R¹³ OH, OC₁₋₄-Alkyl oder N(R⁹)₂ ist.

8. Verbindung nach irgendeinem der vorhergehenden Ansprüche, worin R⁵ H oder C₁₋₆-Alkyl ist.

9. Verbindung nach Anspruch 8, worin:
R¹ Alkyl, Alkenyl, Alkylaryl, Aryl oder Alkyl-AR⁹ ist und R⁹ Alkyl, Aryl oder Heteroaryl ist;
R⁷ H oder R¹⁰CO ist, wobei R¹⁰ Alkyl, Alkylaryl, Cycloalkyl, Cycloalkylalkyl, Alkenyl oder Alkenylaryl ist;
R⁸ gegebenenfalls substituiertes Aryl, Heteroaryl, Alkylaryl, Cycloalkyl, Cycloalkenyl oder Alkylcycloalkyl, Alkyl-R¹¹ oder irgendeine der drei genannten Gruppen ist;
R⁶ Cycloalkyl, Cycloalkenyl, Alkyl, Benzyl, Alkoxybenzyl, Benzyloxybenzyl oder 3-Indolylmethyl ist;
R¹¹ SR⁷, SR⁹, COR¹³, N(R⁹)₂, NR⁹R¹², OR⁹, Succinimido oder Phthalimido ist; und
R¹³ OH, O-Alkyl, O-Alkylaryl, N(R⁹)₂, Alkyl, Aryl oder Alkylaryl ist.

10. Verbindung nach Anspruch 1, ausgewählt aus
*N*-[2,3-bis-Acetylmercaptopropanoyl]-*L*-leucyl-*L*-phenylalanin-*N*-methylamid,
*N*-[2-Acetylmercapto-3-methoxycarbonylpropanoyl]-*L*-leucyl-*L*-phenylalanin-*N*-methylamid,
*N*-[2-Acetylmercapto-4-methoxycarbonylbutanoyl]-*L*-leucyl-*L*-phenylalanin-*N*-methylamid,
*N*-[2-Acetylmercapto-5-methoxycarbonylpentanoyl]-*L*-leucyl-*L*-phenylalanin-*N*-methylamid,
*N*-[2-Acetylmercapto-6-methoxycarbonylhexanoyl]-*L*-leucyl-*L*-phenylalanin-*N*-methylamid,
*N*-[2-Acetylmercapto-4-phthalimidobutanoyl]-*L*-leucyl-*L*-phenylalanin-*N*-methylamid,
*N*-[2-Acetylmercapto-5-phthalimidopentanoyl]-*L*-leucyl-*L*-phenylalanin-*N*-methylamid,
*N*-[2-Acetylmercapto-6-phthalimidohexanoyl]-*L*-leucyl-*L*-phenylalanin-*N*-methylamid,
*N*-[2, 3-bis-Mercaptopropanoyl]-*L*-leucyl-*L*-phenylalanin-*N*-methylamid,
*N*-[2-Mercapto-3-methoxycarbonylpropanoyl]-*L*-leucyl-*L*-phenylalanin-*N*-methylamid,
*N*-[2-Mercapto-4-methoxycarbonylbutanoyl]-*L*-leucyl-*L*-phenylalanin-*N*-methylamid,
*N*-[2-Mercapto-5-methoxycarbonylpentanoyl]-*L*-leucyl-*L*-phenylalanin-*N*-methylamid,
*N*-[2-Mercapto-6-methoxycarbonylhexanoyl]-*L*-leucyl-*L*-phenylalanin-*N*-methylamid,
*N*-[2-Mercapto-4-phthalimidobutanoyl]-*L*-leucyl-*L*-phenylalanin-*N*-methylamid,
*N*-[2-Mercapto-5-phthalimidopentanoyl]-*L*-leucyl-*L*-phenylalanin-*N*-methylamid und
*N*-[2-Mercapto-6-phthalimidohexanoyl]-*L*-leucyl-*L*-phenylalanin-*N*-methylamid.

11. Verbindung nach Anspruch 1, ausgewählt aus
*N*-[2-Acetylmercapto-5-methoxycarbonylpentanoyl]-*L*-leucyl-*L*-phenylalanin-*N*-methylamid,
*N*-[2-Acetylmercapto-6-methoxycarbonylhexanoyl]-*L*-leucyl-*L*-phenylalanin-*N*-methylamid,
*N*-[2-Acetylmercapto-6-methoxycarbonylhexanoyl]-*L*-valinyl-*L*-phenylalanin-*N*-methylamid,
*N*-[2-Acetylmercapto-6-methoxycarbonylhexanoyl]-*L*-leucyl-*L*-tryptophan-*N*-methylamid,
*N*-[2-Acetylmercapto-5-phthalimidopentanoyl]-*L*-leucyl-*L*-phenylalanin-*N*-methylamid,
*N*-[2-Acetylmercapto-5-phthalimidopentanoyl]-*L*-valinyl-*L*-phenylalanin-*N*-methylamid,
*N*-[2-Acetylmercapto-5-phthalimidopentanoyl]-*L*-leucyl-*L*-tryptophan-*N*-methylamid,
*N*-[2-Acetylmercapto-5-phthalimidopentanoyl]-*L*-leucyl-*L*-[β-(4-thiazolyl)]-alanin-*N*-methylamid,
*N*-[2-Acetylmercapto-5-phthalimidopentanoyl]-*L*-leucyl-*L*-[β-(2-pyridyl)]alanin-*N*-methylamid,
*N*-[2-Acetylmercapto-5-phthalimidopentanoyl]-*L*-leucyl-*L*-5-methyl-*L*-glutaminsäure-*N*-methylamid,
*N*-[2-Acetylmercapto-6-phthalimidohexanoyl]-*L*-leucyl-*L*-phenylalanin-*N*-methylamid,
*N*-[2-Acetylmercapto-2-(3-phthalimido)phenylacetyl]-*L*-leucyl-*L*-phenylalanin-*N*-methylamid,
*N*-[2-Mercapto-5-methoxycarbonylpentanoyl]-*L*-leucyl-*L*-phenylalanin-*N*-methylamid,
*N*-[2-Mercapto-6-methoxycarbonylhexanoyl]-*L*-leucyl-*L*-phenylalanin-*N*-methylamid,
*N*-[2-Mercapto-6-methoxycarbonylhexanoyl]-*L*-leucyl-*L*-tryptophan-*N*-methylamid,
*N*-[2-Mercapto-5-phthalimidopentanoyl]-*L*-leucyl-*L*-phenylalanin-*N*-methylamid,
*N*-[2-Mercapto-5-phthalimidopentanoyl]-*L*-leucyl-*L*-tryptophan-*N*-methylamid,
*N*-[2-Mercapto-5-phthalimidopentanoyl]-*L*-leucyl-*L*-[β-(4-thiazolyl)]alanin-*N*-methylamid,
*N*-[2-Mercapto-5-phthalimidopentanoyl]-*L*-leucyl-*L*-[ß-(2-pyridyl)]alanin-*N*-methylamid,
*N*-[2-Mercapto-5-phthalimidopentanoyl]-*L*-leucyl-5-methyl-*L*-glutaminsäure-*N*-methylamid und
*N*-[2-Mercapto-6-phthalimidohexanoyl]-*L*-leucyl-*L*-phenylalanin-*N*-methylamid.

12. Verbindung nach irgendeinem der Ansprüche 1 bis 9, worin R¹ -CH₂SCH₃ ist.

13. Verbindung nach Anspruch 12, ausgewählt aus
(*RS*)-*N*-[2-(Acetylmercapto)-5-phthalimidopentanoyl]-(S-methyl)-*L*-cysteinyl-*L*-phenylalanin-*N*-methylamid,
(*S*)-*N*-[2-Mercapto-5-phthalimidopentanoyl]-*L*-(S-methyl)-*L*-cysteinyl-*L*-tryptophan-*N*-methylamid,
(*RS*)-*N*-[2-Mercapto-4-succinimidobutanoyl]-*L*-(*S*-methyl)cysteinyl-*L*-*tert*-leucin-*N*-methylamid,
(*RS*)-*N*-[2-Mercapto-4-succinimidobutanoyl]-*L*-(*S*-methyl)cysteinyl-*L*-tryptophan-*N*-methylamid und
(*RS*)-*N*-[2-Mercapto4-succinimidobutanoyl]-*L*-(*S*-methyl)cysteinyl-*L*-phenylalanin-*N*-methylamid.

14. Verbindung nach irgendeinem der Ansprüche 1 bis 9, worin R³ tert.-Butyl oder C(CH₃)₂5(O)₀₋₂CH₃ ist.

15. Verbindung nach Anspruch 14, ausgewählt aus
(*S*)-*N*-[2-(Acetylmercapto)-5-phthalimido]pentanoyl-*L*-leucyl-(*S*)-*tert*-leucin-*N*-methylamid,
(*RS*)-*N*-[2-(Acetylmercapto)-5-phthalimido]pentanoyl-*L*-leucyl-(*R*)-penicillamin-*N*-methylamid,
(*RS*)-*N*-[2-(Acetylmercapto)-5-phthalimido]pentanoyl-*L*-leucyl-(*R*)-(*S*-methyl)-penicillamin-*N*-methylamid,
(*RS*)-*N*-[2-(Acetylmercapto)-5-phthalimido]pentanoyl-*L*-leucyl-(*R*)-(2,2-dimethyl-2-methansulfonyl)alanin-*N*-methylamid,
(*RS*)-*N*-[2-(Acetylmercapto)-5-phthalimido]pentanoyl-*L*-leucyl-(*R*)-(2,2-dimethyl-2-methansulfinyl)alanin-*N*-methylamid,
(*RS*)-*N*-[2-(Acetylmercapto)-5-phthalimido]pentanoyl-*L*-leucyl-(*S*)-*tert*-leucin-*N*-methylamid,
(*RS*)-*N*-[2-Mercapto-5-phthalimido]pentanoyl-*L*-leucyl-(*R*)-(*S*-methyl)-penicillamin-*N*-methylamid,
(*S*)-*N*-[2-Mercapto-5-phthalimido]pentanoyl-*L*-leucyl-(*S*)-*tert*-leucin-*N*-methylamid und
(*RS*)-*N*-[2-Mercapto-4-succinimidobutanoyl]-*L*-leucyl-*L*-*tert*-leucin-*N*-methylamid.

16. Verbindung nach irgendeinem der vorhergehenden Ansprüche, in Form eines einzelnen Enantiomers oder Diastereomers oder einer Mischung solcher Isomere.

17. Pharmazeutische Zusammensetzung zur therapeutischen Verwendung, umfassend eine Verbindung nach irgendeinem der vorhergehenden Ansprüche und ein(en) pharmazeutisch annehmbares(n) Verdünnungsmittel oder Träger.

18. Verwendung einer Verbindung nach irgendeinem der Ansprüche 1 bis 16 zur Herstellung eines Medikaments zur Behandlung oder Verhinderung eines Zustands, der mit Matrix-Metalloproteinasen assoziiert ist oder durch TNFα vermittelt wird.

19. Verwendung nach Anspruch 18, worin der Zustand aus Krebs, Entzündung und Entzündungskrankheiten, Gewebedegeneration, Zahnbetterkrankung, Augenerkrankung, dermatologischen Störungen, Fieber, kardiovaskulären Effekten, Hämorrhagie, Blutgerinnung und Akutphase-Reaktion, Kachexie und Anorexie, akuter Infektion, HIV-Infektion, Schockzuständen, Transplantat-Wirt-Reaktionen, Autoimmunerkrankung, Reperfusionssyndrom, Meningitis und Migräne ausgewählt ist.

20. Verwendung nach Anspruch 19, worin der Zustand aus Tumorwachstum, Angiogenese, Tumorinvasion und -ausbreitung, Metastasen, maligner Ascites und malignem Pleuraerguß ausgewählt ist.

21. Verwendung nach Anspruch 19, worin der Zustand aus rheumatoider Arthritis, Osteoarthritis, Osteoporose, Asthma, Multipler Sklerose, Neurodegeneration, Alzheimer-Krankheit, Arteriosklerose, Schlaganfall, Vaskulitis, Crohn-Krankheit und ulcerativer Colitis ausgewählt ist.

22. Verwendung nach Anspruch 19, worin der Zustand aus Hornhautgeschwüren, Retinopathie und Heilung einer Operationswunde ausgewählt ist.

23. Verwendung nach Anspruch 19, worin der Zustand aus Psoriasis, atopischer Dermatitis, chronischen Geschwüren und Epidermolysis bullosa ausgewählt ist.

24. Verwendung nach Anspruch 19, worin der Zustand aus Periodontitis oder Gingivitis ausgewählt ist.

## Revendications

1. Composé de formule générale (I): 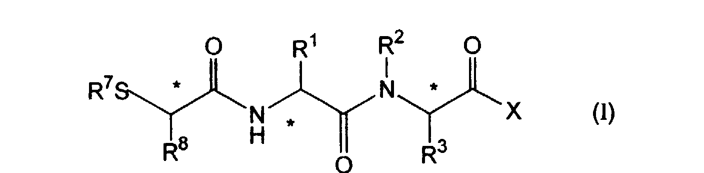 dans laquelle:
R¹ est un groupe alkyle en C₁₋₆, alcényle en C₂₋₆, alkyl(en C₁₋₆)aryle, aryle, alkyl(en C₁₋₆)hétéroaryle, hétéroaryle ou alkyl(en C₁₋₆)-AR⁹ dans lequel A représente O, NR⁹ ou S(O)ₘ dans lequel m = 0 à 2, et R⁹ est H, un groupe alkyle en C₁₋₄, hétéroaryle, alkyl(en C₁₋₄)aryle ou alkyl(en C₁₋₄)hétéroaryle; si A = NR⁹ les groupes R⁹ peuvent être identiques ou différents;
R² est H ou un groupe alkyle en C₁₋₆;
R³ est [Alk]ₙR⁶ dans lequel Alk est un groupe alkyle en C₁₋₆ ou alcényle en C₂₋₆ et n vaut zéro ou 1;
X est NR⁴R⁵ dans lequel R⁴ est l'hydrogène ou un groupe alkyle en C₁₋₆ éventuellement substitué par un groupe amino (NH₂), aryle, arylamino, amino protégé, di(alkyl en C₁₋₆)amine, mono(alkyl en C₁₋₆)amine, CO₂H, carboxyle protégé, carbamoyle, mono(alkyl en C₁₋₆)-carbamoyle ou di(alkyl en C₁₋₆)carbamoyle, et R⁵ est l'hydrogène ou un groupe alkyle en C₁₋₆ ; ou NR⁴R⁵ forme un cycle tel qu'un groupe pyrrolidino, pipéridino ou morpholino;
R⁷ est l'hydrogène ou R¹⁰CO dans lequel R¹⁰ est un groupe alkyle en C₁₋₄, alkyl(en C₁₋₄)aryle, alkyl(en C₁₋₄)hétéroaryle, cycloalkyle en C₃₋₆, alkyl(en C₁₋₄)-cycloalkyle en C₃₋₆, alcényle en C₂₋₆, alcényl(en C₂₋₆)aryle, aryle ou hétéroaryle;
R⁸ est un groupe aryle (substitué par R¹¹), hétéroaryle (éventuellement substitué par R¹¹), alkyle en C₁₋₄-R¹¹, alkyl(en C₁₋₄) aryle(substitué par R¹¹), alkyl(en C₁₋₄)hétéroaryle (éventuellement substitué par R¹¹), cycloalkyle en C₃₋₆ (éventuellement substitué par R¹¹), cycloalcényle en C₃₋₆ (éventuellement substitué par R¹¹), alkyl(en C₁₋₄) cycloalkyle (en C₃₋₆) (éventuellement substitué par R¹¹), ou l'un quelconque des trois groupes 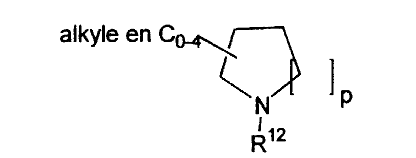 ou 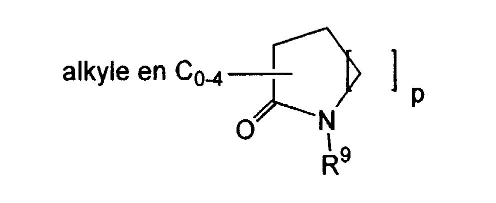 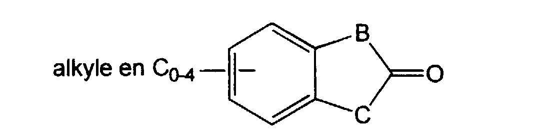 dans lesquels p vaut 1 ou 2 et B et C sont indépendamment choisis parmi O, S C(R⁹)₂ et NR⁹;
R⁶ est AR⁹, un groupe cycloalkyle en C₃₋₆, cycloalcényle en C₃₋₆, alkyle en C₁₋₆, alcoxy(en C₁₋₆)aryle, benzyloxyaryle, aryle, hétéroaryle, alkyl(en C₁₋₃)hétéroaryle, alkyl(en C₁₋₃)aryle, alkyl(en C₁₋₆)-COOR⁹, alkyl (en C₁₋₆)NHR, CONHR, NHCO₂R, NHSO₂R ou NHCOR, R étant défini comme pour R¹⁰;
R¹¹ est SO₂R¹³, SR⁷, SR⁹, COR¹³, N(R⁹)₂, NR⁹R¹², OR⁹, un groupe succinimido ou phtalimido;
R¹² est H ou COR⁹, CO₂R⁹ (dans lesquels R⁹ n'est pas H), CONHR⁹ ou SO₂R⁹ (dans lesquels R⁹ n'est pas H) ; et
R¹³ est OH, un groupe Oalkyle en C₁₋₄, Oalkyl(en C₁₋₄)aryle, N(R⁹)₂ (dans lequel les R⁹ sont identiques ou différents), alkyle en C₁₋₄, aryle, hétéroaryle, alkyle (en C₁₋₄)aryle ou alkyle (en C₁₋₄)hétéroaryle ;
le composé étant sous forme d'un non sel, d'un sel, d'un solvaté ou d'un hydrate.

2. Composé selon la revendication 1, caractérisé en ce que R¹ est un groupe alkyle en C₁₋₆ ou alkyle (en C₁₋₆)AR⁹ dans lequel A est S(O)ₘ, NR⁹, ou O et m = 0, 1 ou 2, et R⁹ est H ou un groupe alkyle en C₁₋₄ ou aryle.

3. Composé selon la revendication 1 ou la revendication 2, caractérisé en ce que R³ est [Alk]ₙR⁶ dans lequel n = 0 ou 1, Alk est un groupe alkyle en C₁₋₆ et R⁶ est un groupe alkyle en C₁₋₆, alkyle (en C₁₋₃)aryle, alkyl (en C₁₋₃) hétéroaryle ou AR⁹.

4. Composé selon l'une quelconque des revendications précédentes, caractérisé en ce que R⁴ est H.

5. Composé selon l'une quelconque des revendications précédentes, caractérisé en ce que X est un groupe pyrrolidino, pipéridino ou morpholino.

6. Composé selon l'une quelconque des revendications précédentes, caractérisé en ce que R⁷ est H ou un groupe alkyl(en C₁₋₆)carbonyle.

7. Composé selon l'une quelconque des revendications précédentes, caractérisé en ce que R⁸ est un groupe alkyl(en C₁₋₄)R¹¹ ou cycloalkyl (en C₃₋₆)R¹¹, et R¹¹ est un groupe COR¹³, NR⁹R¹², N(R⁹)₂, succinimido ou phtalimido, R¹² est un groupe COR⁹, CO₂R⁹ (à condition que R⁹ ne soit pas H), ou SO₂R⁹ (à condition que R⁹ ne soit pas H), et R¹³ est un groupe OH, Oalkyl (en C₁₋₄) ou N(R⁹)₂.

8. Composé selon l'une quelconque des revendications précédentes, caractérisé en ce que R⁵ est H ou un groupe alkyle en C₁₋₆.

9. Composé selon la revendication 8, caractérisé en ce que :
R¹ est un groupe alkyle, alcényle, alkylaryle, aryle ou alktyl-AR⁹ et R⁹ est un groupe alkyle, aryle ou hétéroaryle;
R⁷ est H ou R¹⁰CO dans lequel R¹⁰ est un groupe alkyle, alkylaryle, cycloalkyle, cycloalkylalkyle, alcényle ou alcénylaryle;
R⁸ est un groupe aryle, hétéroaryle, alkylaryle, cycloalkyle, cycloalcényle ou alkylcycloalkyle éventuellement substitué, alkyl-R¹¹ ou l'un quelconque desdits trois groupes ;
R⁶ est un groupe cycloalkyle, cycloalcényle, alkyle, benzyle, alcoxybenzyle, benzyloxybenzyle ou 3-indolylméthyle;
R¹¹ est SR⁷, SR⁹, COR¹³, N(R⁹)₂, NR⁹R¹², OR⁹, un groupe succinimido ou phtalimido;
et
R¹³ est OH, un groupe Oalkyle, Oalkylaryle, N(R⁹)₂, alkyle, aryle ou alkylaryle.

10. Composé selon la revendication 1,choisi parmi
le *N*-méthylamide de la *N*-[2,3-*bis*-acétylmercaptopropanoyl]-*L*-leucyl-*L*-phénylalanine
le *N*-méthylamide de la *N*-[2-acétylmercapto-3-méthoxycarbonylpropanoyl]-*L*-leucyl-*L*-phénylalanine
le *N*-méthylamide de la *N*-[2-acétylmercapto-4-méthoxycarbonylbutanoyl]-*L*-leucyl-*L*-phénylalanine
le *N*-méthylamide de la *N*-[2-acétylmercapto-5-méthoxycarbonylpentanoyl]-*L*-leucyl-*L*-phénylalanine
le *N*-méthylamide de la *N*-[2-acétylmercapto-6-méthoxycarbonylhexanoyl]-*L*-leucyl-*L*-phénylalanine
le *N*-méthylamide de la *N*-[2-acétylmercapto-4-phtalimidobutanoyl]-*L*-leucyl-*L*-phénylalanine
le *N*-méthylamide de la *N*-[2-acétylmercapto-5-phtalimidopentanoyl]-*L*-leucyl-*L*-phénylalanine
le *N*-méthylamide de la *N*-[2-acétylmercapto-6-phtalimidohexanoyl]-*L*-leucyl-*L*-phénylalanine
le *N*-méthylamide de la *N*-[2,3-bis-mercaptopropanoyl]-*L*-leucyl-*L*-phénylalanine
le *N*-méthylamide de la *N*-[2-mercapto-3-méthoxycarbonylpropanoyl]-*L*-leucyl-*L*-phénylalanine
le N-méthylamide de la *N*-[2-mercapto-4-méthoxycarbonylbutanoyl]-*L*-leucyl-*L*-phénylalanine
le *N*-méthylamide de la *N*-[2-mercapto-5-méthoxycarbonylpentanoyl]-*L*-leucyl-*L*-phénylalanine
le *N*-méthylamide de la *N*-[2-mercapto-6-méthoxycarbonylhexanoyl]-*L*-leucyl-*L*-phénylalanine
le *N*-méthylamide de la *N*-[2-mercapto-4-phtalimidobutanoyl]-*L*-leucyl-*L*-phénylalanine
le *N*-méthylamide de la *N*-[2-mercapto-5-phtalimidopentanoyl]-*L*-leucyl-*L*-phénylalanine et
le *N*-méthylamide de la *N*-[2-mercapto-6-phtalimidohexanoyl]-*L*-leucyl-*L*-phénylalanine.

11. Composé selon la revendication 1, choisi parmi
le *N*-méthylamide de la *N*-[2-acétylmercapto-5-méthoxycarbonylpentanoyl]-*L*-leucyl-*L*-phénylalanine
le *N*-méthylamide de la *N*-[2-acétylmercapto-6-méthoxycarbonylhexanoyl]-*L*-leucyl-*L*-phénylalanine
le *N*-méthylamide de la *N*-[2-acétylmercapto-6-méthoxycarbonylhexanoyl]-*L*-valinyl-*L*-phénylalanine
le *N*-méthylamide du *N*-[2-acétylmercapto-6-méthoxycarbonylhexanoyl]-*L*-leucyl-*L*-tryptophane
le *N*-méthylamide de la *N*-[2-acétylmercapto-5-phtalimidopentanoyl]-*L*-leucyl-*L*-phénylalanine
le *N*-méthylamide de la *N*-[2-acétylmercapto-5-phtalimidopentanoyl]-*L*-valinyl-*L*-phénylalanine
le *N*-méthylamide du *N*-[2-acétylmercapto-5-phtalimidopentanoyl]-*L*-leucyl-*L*-trytophane
le *N*-méthylamide de la *N*-[2-acétylmercapto-5-phtalimidopentanoyl]-*L*-leucyl-*L*-[β-(4-thiazolyl)]alanine
le *N*-méthylamide de la *N*-[2-acétylmercapto-5-phtalimidopentanoyl]-*L*-leucyl-*L*-[β-(-2-pyridyl)]alanine
le *N*-méthylamide de l'acide *N*-[2-acétylmercapto-5-phtalimidopentanoyl]-*L*-leucyl-5-méthyl-*L*-glutamique
le *N*-méthylamide de la *N*-[2-acétylmercapto-6-phtalimidohexanoyl]-*L*-leucyl-*L*-phénylalanine
le *N*-méthylamide de la *N*-[2-acétylmercapto-2-(3-phtalimido)-phénylacétyl]-*L*-leucyl-*L*-phénylalanine
le *N*-méthylamide de la *N*-[2-mercapto-5-méthoxycarbonylpentanoyl]-*L*-leucyl-*L*-phénylalanine
le *N*-méthylamide de la *N*-[2-mercapto-6-méthoxycarbonylhexanoyl]-*L*-leucyl-*L*-phénylalanine
le *N*-méthylamide du *N*-[2-mercapto-6-méthoxycarbonylhexanoyl]-*L*-leucyl-*L*-tryptophane
le *N*-méthylamide de la *N*-[2-mercapto-5-phtalimidopentanoyl]-*L*-leucyl-*L*-phénylalanine
le *N*-méthylamide du *N*-[2-mercapto-5-phtalimidopentanoyl]-*L*-leucyl-*L*-tryptophane
le *N*-méthylamide de la *N*-[2-mercapto-5-phtalimidopentanoyl]-*L*-leucyl-*L*-[β(4-thiazolyl)]alanine
le *N*-méthylamide de la *N*-[2-mercapto-5-phtalimidopentanoyl]-*L*-leucyl-*L*-[β (2-pyridyl)]alanine
le *N*-méthylamide de l'acide *N*-[2-mercapto-5-phtalimidopentanoyl]-*L*-leucyl-5-méthyl-*L*-glutamique et
le *N*-méthylamide de la *N*-[2-mercapto-6-phtalimidohexanoyl]-*L*-leucyl-*L*-phénylalanine.

12. Composé selon l'une quelconque des revendications 1 à 9, caractérisé en ce que R¹ est -CH₂SCH₃.

13. Composé selon la revendication 12, choisi parmi
le *N*-méthylamide de la (*RS*)-*N*-[2-(acétylmercapto)-5-phtalimidopentanoyl]-(*S*-méthyl)-*L*-cystéinyl-*L*-phénylalanine
le *N*-méthylamide du (*S*)-*N*-[2-mercapto-5-phtalimidopentanoyl]-*L*-(*S*-méthyl)-cystéinyl-*L*-tryptophane
le *N*-méthylamide de la (*RS*)-*N*-[2-mercapto-4-succinimidobutanoyl]-*L*-(*S*-méthyl)-cystéinyl-*L*-*tert*-leucine
le *N*-méthylamide du (*RS*)-*N*-[2-mercapto-4-succinimidobutanoyl]-*L*-(*S*-méthyl)-cystéinyl-*L*-tryptophane et
le *N*-méthylamide de la (*RS*)-*N*-[2-mercapto-4-succinimidobutanoyl]-*L*-(*S*-méthyl)-cystéinyl-*L*-phénylalanine.

14. Composé selon l'une quelconque des revendications 1 à 9, caractérisé en ce que R³ est un groupe *tert*-butyle ou C(CH₃)₂S(O)₀₋₂CH₃.

15. Composé selon la revendication 14, choisi parmi
le *N*-méthylamide de la (*S*)-*N*-[2-(acétylmercapto)-5-phtalimido]-pentanoyl-*L*-leucyl-(*S*)-*tert*-leucine
le *N*-méthylamide de la (*RS*)-*N*-[2-(acétylmercapto)-5-phtalimido]-pentanoyl-*L*-leucyl-(*R*)-pénicillamine
le *N*-méthylamide de la (*RS*)-*N*-[2-(acétylmercapto)-5-phtalimido]-pentanoyl-*L*-leucyl-(*R*)-(*S*-méthyl)-pénicillamine
le *N*-méthylamide de la (*RS*)-*N*-[2-(acétylmercapto)-5-phtalimido]-pentanoyl-*L*-leucyl-(*R*)-(2,2-diméthyl-2-méthanesulfonyl)alanine
le *N*-méthylamide de la (*RS*)-*N*-[2-(acétylmercapto)-5-phtalimido]-pentanoyl-*L*-leucyl-(*R*)-(2 ,2-diméthyl-2-méthanesulfinyl)alanine
le *N*-méthylamide de la (*RS*)-*N*-[2-(acétylmercapto)-5-phtalimido]-pentanoyl-*L*-leucyl-(*S*)*-tert-*leucine
le *N*-méthylamide de la (*RS*)-*N*-[2-mercapto-5-phtalimido]pentanoyl-*L*-leucyl-(*R*)-(*S*-méthyl)pénicillamine
le *N*-méthylamide de la (*S*)-*N*-[2-mercapto-5-phtalimido]pentanoyl-*L*-leucyl-(*S*)-*tert*-leucine et
le *N*-méthylamide de la (*RS*)-*N*-[2-mercapto-4-succinimidobutanoyl]-*L*-leucyl-*L*-*tert*-leucine.

16. Composé selon l'une quelconque des revendications précédentes, sous la forme d'un énantiomère ou diastéréomère unique, ou un mélange de tels isomères.

17. Composition pharmaceutique pour un usage en thérapie, comprenant un composé selon l'une quelconque des revendications précédentes, et un diluant ou un support acceptables du point de vue pharmaceutique.

18. Utilisation d'un composé selon l'une quelconque des revendications 1 à 16, pour la fabrication d'un médicament pour le traitement ou la prévention d'un état associé avec la matrice de métalloprotéinases ou qui est induit par TNFα.

19. Utilisation selon la revendication 18, caractérisée en ce que l'état est chois parmi le cancer, l'inflammation et des maladies inflammatoires, une dégénérescence des tissus, la périodontite, une maladie ophtalmologique, des désordres en dermatologie, la fièvre, des effets cardio-vasculaires, des hémorragies, la coagulation ou une réponse à une phase aiguë, la cachexie et l'anorexie, une infection aiguë, une infection par HIV, des états de choc, des réactions d'hôtes par rapport à des parasites, des maladies auto-immunes, une blessure par perfusion, la méningite et la migraine.

20. Utilisation selon la revendication 19, caractérisée en ce que l'état est choisi parmi une croissance tumorale, l'angéite, une invasion et l'accroissement de tumeur, des métastases, des ascites malins et une effusion pleurale maligne.

21. Utilisation selon la revendication 19, caractérisée en ce que l'état est choisi parmi l'arthrite rhumatoïde, l'ostéo-arthrite, l'ostéoporose, l'asthme, la sclérose multiple, la neuro-dégénérescence, la maladie d'Alzheimer, l'artériosclérose, les coups, la vascularite, la maladie de Crohn et la colite ulcéreuse.

22. Utilisation selon la revendication 19, caractérisée en ce que l'état est choisi parmi l'ulcération de la cornée, la rétinopathie et la cicatrisation de blessure opératoire.

23. Utilisation selon la revendication 19, caractérisée en ce que l'état est choisi parmi le psoriasis, la dermatite atopique, les ulcères chroniques et l'épidermolyse bulleuse.

24. Utilisation selon la revendication 19, caractérisée en ce que l'état est choisi parmi la périodontite ou la gingivite.
